# EUROPEAN PATENT APPLICATION

(11) **EP 4 659 668 A1**
(43) Date of publication of application: **10.12.2025**
(21) Application number: 25174861.2
(22) Date of filing: 07.05.2025
(51) Int. Cl.: A61B 5/11, A61B 5/00, A61N 1/365

(54) **METHODS AND SYSTEMS FOR HEART HEALTH DIAGNOSTICS**

(30) Priority: 05.06.2024 US 202463656257 P; 05.05.2025 US 202519198813
(71) Applicant: Pacesetter, Inc., Sylmar, CA 91342 (US)
(72) Inventor: Mouchawar, Gabriel A., Sylmar, CA 91342 (US); Gill, Jong, Sylmar, CA 91342 (US); Dawoud, Fady, Sylmar, CA 91342 (US); Qu, Fujian, Sylmar, CA 91342 (US)
(74) Representative: Barker Brettell Sweden AB

(57) **Abstract**

A system (1000) for monitoring a physiologic condition of a patient. The system (1000) comprising an implantable medical device (IMD) (100, 700, 1003) comprising an accelerometer (112, 770, 1030) configured to output an accelerometer signal, sensing circuitry (744) configured to sense a cardiac activity (CA) signal, and a memory (760) configured to store program instructions. One or more processors (721) coupled to the accelerometer (112, 770, 1030) and sensing circuitry (744) that, when executing the program instructions, are configured to: determine a heart rate (HR) at a point in time based on the CA signal; determine an activity level (AL) at the point in time based on accelerometer data, the accelerometer data based on the accelerometer signal, the HR and AL forming an HR-AL event; compare the HR-AL event to a heart condition (HC) metric that defines combinations of HRs and ALs representing a physiologically normal heart condition and a physiologically abnormal heart condition; and identify a physiologically abnormal heart condition based on the comparison.

## Description

### TECHNICAL FIELD

Embodiments herein generally relate to systems for detecting a health condition of a heart by evaluating heart rate dynamics at various activity levels using an implantable medical device implanted within a patient.

### BACKGROUND

Insertable cardiac monitors (ICMs) have been increasingly used to diagnose and monitor a variety of heart conditions and guide clinical management in patients at high risk of cardiac arrhythmias. Conventional ICMs are generally programmed to detect a particular type of arrhythmia and to only save electrogram/electrocardiogram (EGM/ECG) signals and/or markers associated with the particular arrhythmia of interest. For example, ICMs have been proposed to monitor heart signals for atrial fibrillation, pause episodes or other specific types of arrhythmias. When an episode of interest occurs, the ICM records the EGM/ECG signals and/or related markers that occurred during the corresponding episode.

ICMs, as well as all implantable medical devices, have limited memory storage capacity. This constrains the design of the device to only save data of importance to the specific arrhythmia of interest. Many ICMs rely on a trigger to acquire data, potentially missing useful information (preceding an episode) that can be used to monitor and diagnose a patient's condition.

Some ICMs include a three-dimensional (3-D) accelerometer that is configured to detect movement of the patient during day-to-day activities. The 3-D accelerometer can also detect a patient's body posture, such as by detecting rotation based on the position and/or orientation of the ICM.

A need exists for improvements in devices and systems to monitor a patient's heart condition and capture information that is related to an unhealthy heart condition but is not necessarily related to a specific type of arrhythmia. A need further exists for metrics and displays that convey meaningful information to healthcare providers to articulate the relationship between healthy and unhealthy heart conditions versus combinations of heart rates, activity levels, and/or posture.

### BRIEF SUMMARY

In accordance with embodiments herein, a system comprises an implantable medical device (IMD) configured to be implanted in the patient, the IMD comprising: an accelerometer configured to output an accelerometer signal; sensing circuitry configured to sense a cardiac activity (CA) signal over a plurality of cardiac cycles; memory configured to store program instructions; and one or more processors coupled to the accelerometer and the sensing circuitry. The one or processors, when executing the program instructions, are configured to: determine a heart rate (HR) at a point in time based on the CA signal; determine an activity level (AL) at the point in time based on accelerometer data, the accelerometer data based on the accelerometer signal, the HR and AL forming an HR-AL event; compare the HR-AL event to a heart condition (HC) metric that defines combinations of HRs and ALs representing a physiologically normal heart condition and at least one physiologically abnormal heart condition; and identify a physiologically abnormal heart condition based on the comparison.

Optionally, the one or more processors are further configured to repeat the determining and comparing operations to build an HC assessment data set; save the HC assessment data set in the memory of the IMD, and transmit the HC assessment data set to an external device or a second IMD configured to be implanted in the patient, the HC assessment data set utilized to identify the physiologically abnormal heart condition.

Optionally, the second IMD is configured to deliver therapy to treat the physiologically abnormal heart condition identified by the HC assessment data set.

Optionally, the IMD further comprises a pulse generator configured to generate electrical pulses to be applied to the patient's heart, wherein the one or more processors are, when executing the program instructions, configured to control the pulse generator to generate pacing pulses to treat the identified physiologically abnormal heart condition.

Optionally, the external device is configured to receive the HC assessment data set and map the HC assessment data set onto an HR-AL template to form a HC treatment profile, the external device having a display configured to display a graphical representation of the HC treatment profile.

Optionally, the HC treatment profile is divided into activity zones for i) no activity, ii) low activity, iii) moderate activity, and iv) high activity, and further divided into HR zones for i) low HR, ii) medium HR, and iii) high HR, the HC assessment data set mapped into the activity and HR zones.

Optionally, the HC treatment profile indicates an increase in a number of the HR-AL events at a relatively higher HR over at least two activity levels.

Optionally, the HC metric includes upper and lower HC boundaries, wherein a range between the upper and lower HC boundaries corresponds to physiologically healthy HR-AL events, and the regions above the upper HC boundary and below the lower HC boundary correspond to physiologically unhealthy HR-AL events.

Optionally, the one or more processors are further configured to delete the HR-AL events that fall within a range of the HC metric corresponding to the combinations of HRs and ALs representing the physiologically normal heart condition such that the HC assessment data set excludes the HR-AL events that fall within the range.

Optionally, the one or more processors are further configured to save, in the HC assessment data set, the HR-AL events that fall in regions of the HC metric corresponding to the combinations of HRs and ALs representing the physiologically abnormal heart condition.

Optionally, wherein in response to the physiologically abnormal heart condition being identified, the HC assessment data set prescribes treatment for at least one of i) chronotropic incompetence, ii) sick sinus syndrome, iii) heart failure, iv) sleep apnea, or v) fitness level of the patient.

Optionally, the HC assessment data set prescribes no treatment when the HC assessment data set is indicative of the physiologically normal heart condition.

Optionally, the one or more processors are further configured to determine, using the accelerometer data, a posture from within a plurality of postures over the monitoring period; and wherein each of the HR-AL events saved in the HC assessment data set in the memory is associated with the corresponding posture at the corresponding point in time.

Optionally, the HC assessment data set excludes at least a portion of the HR-AL events associated with at least one of the postures.

Optionally, wherein the one or more processors is further configured to transmit the HR-AL event or a result of the comparison to a second device.

Optionally, wherein the IMD further comprises a transceiver configured to transmit the HR-AL event or a result of the comparison to a second device.

In accordance with embodiments herein a system for monitoring a physiologic condition of a patient, the system comprising an implantable medical device (IMD) configured to be implanted in the patient, the IMD comprising an accelerometer configured to output an accelerometer signal; sensing circuitry configured to sense a cardiac activity (CA) signal over a plurality of cardiac cycles; memory configured to store program instructions; and one or more processors. The one or more processors are coupled to the accelerometer and the sensing circuitry, and when executing the program instructions, are configured to: determine a heart rate (HR) at a point in time based on the CA signal; determine an activity level (AL) at the point in time based on accelerometer data, the accelerometer data based on the accelerometer signal, the HR and AL forming an HR-AL event; compare the HR-AL event to a heart condition (HC) metric that defines combinations of HRs and ALs representing a physiologically normal heart condition and a physiologically abnormal heart condition; and transmit the HR-AL event or a result of the comparison to a second device, the HR-AL event or the result of the comparison utilized to treat a heart condition.

The various embodiments described in the foregoing also apply to the system as defined above.

### BRIEF DESCRIPTION OF THE DRAWINGS

Figure 1 illustrates an example implantable medical device (IMD) intended for subcutaneous implantation at a site near the heart that may include an accelerometer in accordance with embodiments herein.
Figure 2 illustrates a method for collecting heart rate and activity level (HR-AL) events indicative of a physiologic condition of a patient to determine a relationship between heart rate and activity and/or posture in accordance with embodiments herein.
Figure 3 illustrates a heart condition metric utilized in accordance with embodiments herein.
Figure 4A illustrates a graphical representation for an HC treatment profile of normal sinus rhythm (NSR) that can be measured in relatively healthy patients in accordance with embodiments herein.
Figure 4B illustrates a graphical representation of an HC treatment profile for a patient experiencing chronotropic incompetence in accordance with embodiments herein.
Figure 4C illustrates a graphical representation of an HC treatment profile for a patient experiencing supraventricular tachycardia in accordance with embodiments herein.
Figure 4D illustrates a graphical representation of an HC treatment profile for a patient experiencing heart failure in accordance with embodiments herein.
Figure 4E illustrates a graphical representation for an HC treatment profile of a patient experiencing sick sinus syndrome in accordance with embodiments herein.
Figure 4F illustrates a graphical representation of an HC treatment profile used to assess and track the progress of cardiac fitness in accordance with embodiments herein.
Figure 5 illustrates a method for monitoring a physiologic condition, determining a treatment and delivering the treatment in accordance with embodiments herein.
Figure 6 illustrates a method for interfacing with a patient application in accordance with embodiments herein.
Figure 7 shows a block diagram of the IMD formed in accordance with embodiments herein.
Figure 8 illustrates a schematic diagram of a digital healthcare system in accordance with embodiments herein.
Figure 9 illustrates a high-level flowchart of a method, implemented by the medical network of Figure 8, for processing current or real-time HR data, accelerometer data and/or IMD data in accordance with embodiments herein.
Figure 10 illustrates a schematic diagram of a healthcare system in accordance with embodiments herein.

### DETAILED DESCRIPTION

The terms "heart condition boundary" and "HC boundary" shall refer to a relationship between heart rate and activity level that extends over a range of heart rates and a range of activity levels. For example, each detected or measured heart rate has an associated activity level.

The term "ASM" shall mean application-specific model.

The terms "beat" and "cardiac event" are used interchangeably and refer to both normal and/or abnormal events.

The terms "cardiac activity signal", "cardiac activity signals", "CA signal" and "CA signals" (collectively "CA signals") are used interchangeably throughout to refer to measured signals indicative of cardiac activity for the heart or more specifically for a region or chamber of interest. For example, the CA signals may be indicative of impedance, electrical or mechanical activity of the heart overall or more specifically, of one or more chambers (e.g., left or right ventricle, left or right atrium) of the heart and/or of a local region within the heart (e.g., impedance, electrical or mechanical activity at the AV node, along the septal wall, within the left or right bundle branch, within the purkinje fibers). The cardiac activity may be normal/healthy or abnormal/arrhythmic. An example of CA signals includes EGM signals. Electrical based CA signals refer to an analog or digital electrical signal recorded by two or more electrodes, where the electrical signals are indicative of cardiac activity. Heart sound (HS) based CA signals refer to signals output by a heart sound sensor, such as an accelerometer, where the HS based CA signals are indicative of one or more of the S1, S2, S3 and/or S4 heart sounds. Impedance based CA signals refer to impedance measurements recorded along an impedance vector between two or more electrodes, where the impedance measurements are indicative of cardiac activity.

The term "health care system" references to a system that includes equipment for measuring health parameters, and communication pathways from the equipment to secondary devices. The secondary devices may be at the same location as the equipment, or remote from the equipment at a different location. The communication pathways may be wired, wireless, over the air, cellular, in the cloud, etc. In one example, the healthcare system provided may be one of the systems described in U.S. published application US20210020294A1 entitled METHODS DEVICE AND SYSTEMS FOR HOLISTIC INTEGRATED HEALTHCARE PATIENT MANAGEMENT, filed July 16, 2020. Other patents that describe example monitoring systems include U.S. Pat. No. 6,572,557 entitled SYSTEM AND METHOD FOR MONITORING PROGRESSION OF CARDIAC DISEASE STATE USING PHYSIOLOGIC SENSORS, filed Dec. 21, 2000; U.S. Pat. No. 6,480,733 entitled METHOD FOR MONITORING HEART FAILURE filed Dec. 17, 1999; U.S. Pat. No. 7,272,443 entitled SYSTEM AND METHOD FOR PREDICTING A HEART CONDITION BASED ON IMPEDANCE VALUES USING AN IMPLANTABLE MEDICAL DEVICE, filed Dec. 14, 2004; U.S. Pat. No. 7,308,309 entitled DIAGNOSING CARDIAC HEALTH UTILIZING PARAMETER TREND ANALYSIS, filed Jan. 11, 2005; and U.S. Pat. No. 6,645,153 entitled SYSTEM AND METHOD FOR EVALUATING RISK OF MORTALITY DUE TO CONGESTIVE HEART FAILURE USING PHYSIOLOGIC SENSORS, filed Feb. 7, 2002.

Additionally or alternatively, embodiments herein may be implemented in connection with the methods and systems described in US Published Application 2021/0345891 titled "METHOD AND DEVICE FOR DETECTING RESPIRATION ANOMALY FROM LOW FREQUENCY COMPONENT OF ELECTRICAL CARDIAC ACTIVITY SIGNALS", filed May 8, 2020, and US Published Application 2021/0345935 titled "SYSTEM FOR VERIFYING A PATHOLOGIC EPISODE USING AN ACCELEROMETER".

The term "IMD" shall mean an implantable medical device. Embodiments may be implemented in connection with one or more implantable medical devices (IMDs). Non-limiting examples of IMDs include one or more of neurostimulator devices, implantable leadless monitoring and/or therapy devices, and/or alternative implantable medical devices. For example, the IMD may represent a cardiac monitoring device, pacemaker, cardioverter, cardiac rhythm management device, defibrillator, neurostimulator, leadless monitoring device, leadless pacemaker and the like. The IMD may measure electrical and/or mechanical information. For example, the IMD may include one or more structural and/or functional aspects of the device(s) described in U.S. Patent Number 9,333,351, entitled "NEUROSTIMULATION METHOD AND SYSTEM TO TREAT APNEA" issued May 10, 2016 and U.S. Patent Number 9,044,610, entitled "SYSTEM AND METHODS FOR PROVIDING A DISTRIBUTED VIRTUAL STIMULATION CATHODE FOR USE WITH AN IMPLANTABLE NEUROSTIMULATION SYSTEM" issued June 02, 2015. The IMD may monitor transthoracic impedance, such as implemented by the CorVue algorithm offered by St. Jude Medical. Additionally or alternatively, the IMD may include one or more structural and/or functional aspects of the device(s) described in U.S. Patent Number 9,216,285, entitled "LEADLESS IMPLANTABLE MEDICAL DEVICE HAVING REMOVABLE AND FIXED COMPONENTS" issued December 22, 2015 and U.S. Patent Number 8,831,747, entitled "LEADLESS NEUROSTIMULATION DEVICE AND METHOD INCLUDING THE SAME" issued September 09, 2014. Additionally or alternatively, the IMD may include one or more structural and/or functional aspects of the device(s) described in U.S. Patent Number 8,391,980, entitled "METHOD AND SYSTEM FOR IDENTIFYING A POTENTIAL LEAD FAILURE IN AN IMPLANTABLE MEDICAL DEVICE" issued March 05, 2013 and U.S. Patent Number 9,232,485, entitled "SYSTEM AND METHOD FOR SELECTIVELY COMMUNICATING WITH AN IMPLANTABLE MEDICAL DEVICE" issued January 05, 2016. Additionally or alternatively, the IMD may be a subcutaneous IMD that includes one or more structural and/or functional aspects of the device(s) described in U.S. Patent Number 10,765,860, entitled "SUBCUTANEOUS IMPLANTATION MEDICAL DEVICE WITH MULTIPLE PARASTERNAL-ANTERIOR ELECTRODES" filed May 07, 2018; U.S. Patent Number 10,722,704, entitled "IMPLANTABLE MEDICAL SYSTEMS AND METHODS INCLUDING PULSE GENERATORS AND LEADS" filed May 07, 2018; U.S. Patent Number 11,045,643, entitled "SINGLE SITE IMPLANTATION METHODS FOR MEDICAL DEVICES HAVING MULTIPLE LEADS", filed May 07, 2018. Further, one or more combinations of IMDs may be utilized from the above mentioned patents and applications in accordance with embodiments herein. Embodiments may be implemented in connection with one or more subcutaneous implantable medical devices (S-IMDs). For example, the S-IMD may include one or more structural and/or functional aspects of the device(s) described in U.S. Patent Number 10,722,704, entitled "IMPLANTABLE MEDICAL SYSTEMS AND METHODS INCLUDING PULSE GENERATORS AND LEADS", filed May 07, 2018; U.S. Patent Number 10,765,806, entitled "SUBCUTANEOUS IMPLANTATION MEDICAL DEVICE WITH MULTIPLE PARASTERNAL-ANTERIOR ELECTRODES". The IMD may represent a passive device that utilizes an external power source and/or an active device that includes an internal power source. The IMD may deliver some type of therapy/treatment, provide mechanical circulatory support and/or merely monitor one or more physiologic characteristics of interest (e.g., pulmonary arterial pressure ("PAP"), CA signals, impedance, heart sounds). Additionally, or alternatively, embodiments may be implemented in connection with one or more passive IMDS (PIMDs). Non-limiting examples of PIMDs may include passive wireless sensors used by themselves, or incorporated into or used in conjunction with other IMDs, such as cardiac monitoring devices, pacemakers, cardioverters, cardiac rhythm management devices, defibrillators, neurostimulators, leadless monitoring devices, leadless pacemakers, replacement valves, shunts, grafts, drug elution devices, blood glucose monitoring systems, orthopedic implants, and the like. For example, embodiments may implement one or more structural and/or functional aspects of the device(s) described in U.S. Patent No. 9,265,428 entitled "Implantable Wireless Sensor", U.S. Patent No. 8,278,941 entitled "Strain Monitoring System and Apparatus", U.S. Patent No. 8,026,729 entitled "System and Apparatus for In-Vivo Assessment of Relative Position of an Implant", U.S. Patent No. 8,870,787 entitled "Ventricular Shunt System and Method", and U.S. Patent No. 9,653,926 entitled "Physical Property Sensor with Active Electronic Circuit and Wireless Power and Data Transmission"

The term "chronotropic incompetence" or "CI", shall mean an inability of the heart to increase its rate commensurate with increased activity or demand. CI is common in patients with cardiovascular disease, produces exercise intolerance that impairs quality of life, and is an independent predictor of major adverse cardiovascular events and overall mortality.

The term "external device" shall mean an electronic device that is configured 1) to receive patient data that is entered by the patient, such as via a user interface, and/or 2) to receive, optionally process, and in some cases, display patient data in connection with an IMD and an accelerometer. The external device may include, but is not limited to, smart phones, desktop or laptop computers, tablet devices, smart TVs, fixed cameras, smart watch, wearable heart rate monitor, portable or handheld cameras, recording devices, digital personal assistant (DPA) devices, readers, programmers, external computing devices, patient data entry (PDA) devices, and the like. In addition, the external device may represent various types of devices configured to record audio and/or voice signatures, detect gestures and movements and the like. The external device may include a graphical user interface, through which the patient or another user enters the patient data. Optionally, the external device may include audio and/or video sensors/cameras that may receive patient data. For example, a user may use a keyboard, touch screen and/or mouse, or microphone to enter patient data.

The term "obtains" and "obtaining", as used in connection with data, signals, information and the like, include at least one of i) accessing memory of an external device or remote server where the data, signals, information, etc., are stored, ii) receiving the data, signals, information, etc., over a wireless communications link between the IMD and a local external device, and/or iii) receiving the data, signals, information, etc., at a remote server over a network connection. The obtaining operation, when from the perspective of an IMD, may include sensing new signals in real-time, and/or accessing memory to read stored data, signals, information, etc., from memory within the IMD. The obtaining operation, when from the perspective of a local external device, includes receiving the data, signals, information, etc., at a transceiver of the local external device where the data, signals, information, etc., are transmitted from an IMD and/or a remote server. The obtaining operation may be from the perspective of a remote server, such as when receiving the data, signals, information, etc., at a network interface from a local external device and/or directly from an IMD. The remote server may also obtain the data, signals, information, etc., from local memory and/or from other memory, such as within a cloud storage environment and/or from the memory of a workstation or clinician external programmer.

The terms "posture" and "patient posture" refer to posture positions of a patient, including a standing posture, sitting posture, supine posture, prone posture, left lateral recumbent (LLR), right lateral recumbent (RLR), recline, incline, 45 degree incline, and the like.

The term "probability" shall mean, not only a determined percentage or numerical value, but also non-numerical values and corresponding indicators. For example, a risk score algorithm may be used to determine a risk range or risk category a patient falls into with the range or risk category illustrated by color, bars, and the like. Such probability may also include morphological comparisons such as comparisons of waveforms in graphs associated with heart related data. In this manner, probability is simply the factoring or use of event related variable(s) to determine the likelihood an event will or will not occur. The probability thus may be represented in numerous manners, including a risk score, a subsequent risk score, a percentage, a bar graph, a range, a color-coded indicator, text indicia providing an indicator text such as "low", "medium", and "high", pictorially, and the like.

The terms "processor," "a processor", "one or more processors" and "the processor" shall mean one or more processors. The one or more processors may be implemented by one, or by a combination of more than one implantable medical device, a wearable device, a local device, a remote device, a server computing device, a network of server computing devices and the like. The one or more processors may be implemented at a common location or at distributed locations. The one or more processors may implement the various operations described herein in a serial or parallel manner, in a shared-resource configuration and the like.

The term "real-time" shall refer to a time period substantially contemporaneous with an event of interest. The term "real-time," when used in connection with obtaining and/or processing data utilizing an IMD, shall refer to processing operations performed substantially contemporaneous with a physiologic event of interest experienced by a patient. By way of example, in accordance with embodiments herein, physiologic and accelerometer signals are analyzed in real time (e.g., during a cardiac event, while walking/running at a particular speed, during a prescribed physical patient activity or within a few minutes after the cardiac event or prescribed physical patient activity).

The term "sick sinus syndrome" or "SSS", also known as sinus node dysfunction, shall mean a disorder of the sinoatrial node caused by impaired SA note function and impulse transmission producing a constellation of abnormal rhythms. These include atrial brady-arrhythmias, atrial tachyarrhythmias and, sometimes, bradycardia alternating with tachycardia often referred to as tachy-brady syndrome. These arrhythmias may result in palpitations and decreased tissue perfusion and consequent fatigue, lightheadedness, pre-syncope, and syncope.

The terms "treat" and "treatment", when used in connection with a heart condition, shall mean to affect a particular treatment or prophylaxis for a heart disease or heart condition, including i) to prevent a particular heart disease or heart condition, ii) to change (e.g., slow) progression of the particular heart disease or heart condition, and/or iii) to monitor and/or analyze the particular heart disease or heart condition. By way of example, the treatment may constitute i) delivering a stimulation therapy or drug by an IMD, a wearable medical device, or an external device, ii) changing in a stimulation parameter or drug regiment, iii) prescribing implant of an IMD, iv) prescribing a drug delivery pump, v) implanting an IMD or drug delivery pump, and/or vi) recommending increasing/decreasing activity. For the avoidance of doubt, the treatment shall include prescribing implant and/or actual implant of an IMD to delivery stimulation therapy, programming stimulation parameters of the IMD, and/or delivering stimulation therapy by the IMD to treat a heart condition such as one or more of chronotropic incompetence, sick sinus syndrome, supraventricular tachycardia, and/or heart failure.

### System Overview

Conventional ICMs, and more generally IMDs, are programmed to detect a particular type of arrhythmia and to save only EGM/ECG signals and/or markers associated with episodes for the particular arrhythmia of interest. For example, ICMs have been proposed to monitor heart signals for atrial fibrillation, pause episodes or other specific types of arrhythmias. When an episode of interest occurs, the ICM records the EGM/ECG signals and/or related markers that occurred during the corresponding episode. ICMs, as well as all implantable medical devices, have limited memory storage capacity. This constrains the design of the device to only save data of importance to the specific arrhythmia of interest. As previously noted, many ICMs rely on a trigger to acquire data, potentially missing useful information (preceding an episode) that can be used to monitor and diagnose a patient's condition.

In accordance with new and unique aspects herein, it has been realized that evaluation of heart rate dynamics at various activity levels has treatment, diagnostic and predictive value, separate from implanting an IMD designed to target a specific arrhythmia of interest. Accordingly, new and unique embodiments herein configure an IMD (e.g., ICM) to simultaneously or substantially simultaneously acquire heart rate and activity level (HR-AL) events not previously acquired and to compare the newly acquired HR-AL events to new diagnostic metrics to build heart condition (HC) assessment data sets utilized to identify and/or treat a corresponding heart condition. According to new and unique embodiments herein, optionally the HR-AL event information from the HC assessment data sets are formatted and displayed in a new manner to better illustrate and diagnose a patient's heart health issues (e.g., sick sinus syndrome, chronotropic incompetence, heart failure (HF), fitness level) and their chronic changes. In accordance with new and unique aspects, improvements to computer and patient monitoring technology are provided in a system for monitoring physiologic conditions of a patient. The system comprises an IMD configured to be implanted in the patient. The IMD comprises: an accelerometer configured to output an accelerometer signal; sensing circuitry configured to sense a cardiac activity (CA) signal over a plurality of cardiac cycles; and memory configured to store program instructions. The improvement in the computer and patient monitoring technology includes, among other things, configuring one or more processors coupled to the accelerometer and the sensing circuitry that, when executing the program instructions, to: determine a heart rate (HR) at a point in time based on the CA signal; determine an activity level (AL) at the point in time based on accelerometer data, wherein the accelerometer data is based on the accelerometer signal, the HR and AL pair forming an HR-AL event; compare the HR-AL events to a heart condition (HC) metric that defines combinations of HRs and ALs representing a physiologically normal heart condition and a physiologically abnormal heart condition; and identify a physiologically abnormal heart condition based on the comparison.

In some embodiments, the one or more processors transmit the HR-AL event or a result of the comparison to a second device.

In some embodiments, the one or more processors repeat the determining and comparing operations to build an HC assessment data set and save the HC assessment data set in the memory of the IMD, wherein the memory is coupled to the one or more processors.

Conventional computers and patient monitoring technology do not collect combinations of HR and AL data at corresponding points in time and therefore are unable to form HR-AL events. Conventional computers and patient monitoring technology do not provide heart condition metrics that define combinations of HRs and ALs representing a physiologically normal heart condition and a physiologically abnormal heart condition. Conventional computers and patient monitoring technology do not compare HR-AL events to an HC metric, nor build an HC assessment data set, nor save an HC assessment data set in the IMD and/or external device, nor transmit an HC assessment data set to an external device, nor provide an HC assessment data set to treat the heart condition. By collecting HR-AL events and developing HC metrics corresponding thereto, embodiments herein afford improvements to computers and patient monitoring technology.

Further, IMDs have limited memory capabilities and, thus, cannot save an open ended or endless amount of data over an extended period of time. Accordingly, an additional, or alternative, improvement herein allows the IMD to be programmed to save only HR-AL events of interest, namely only HR-AL events that occur outside of predetermined parameters (e.g., above and/or below a defined healthy range). For example, the IMD can be programmed to acquire HR-AL events that occur outside of heart rate and activity level boundaries for healthy heart conditions. The IMD determines, in an embodiment, which HR-AL events to save in memory utilizing an HC metric having an upper heart rate boundary and a lower heart rate boundary. The range or area between the upper and lower heart rate boundaries represents normal response of the heart to activity level (e.g., physiologically normal heart condition), and HR-AL events detected within this range/area can be discarded, saving memory and battery life. HR-AL events detected outside of the upper and lower heart rate boundaries are saved to build an HC assessment data set which can be transmitted to an external device.

Further, given that conventional computers and patient monitoring technology do not collect HR-AL events, it also follows that they do not offer a format to display HR-AL events collected over a monitoring period in a manner that is efficient and easily understandable to correspond to a particular heart condition. For example, no approach has been suggested to display combinations of heart rate and activity level over a monitoring period to efficiently and easily understand that the data (HC assessment data set) indicates one or more of chronotropic incompetence, sick sinus syndrome, supraventricular tachycardia, and/or heart failure.

In accordance with new and unique aspects herein, HR-AL events are formatted for display in a graphical manner that clearly shows the relationship between heart rate and activity level and can be used to treat (e.g., monitor or diagnose) a physiologically normal or abnormal condition of the patient. In some embodiments, the graphical representations can be displayed as scatter plots. Individual HR-AL events can be displayed, or HR-AL events can be grouped together, such as in a block representing a heart rate range and an activity range. Additionally, or alternatively, the graphical representation of the HC assessment data set, to be displayed, can be divided into activity zones for e.g., i) no activity, ii) low activity, iii) moderate activity, and iv) high activity, and further divided into HR zones for e.g., i) low HR, ii) medium HR, and iii) high HR. Other graphical representations can be used, such as bar graphs, pie charts, etc. Graphical representations can be compared to templates, such as age-related templates, templates representing particular diagnosis, etc.

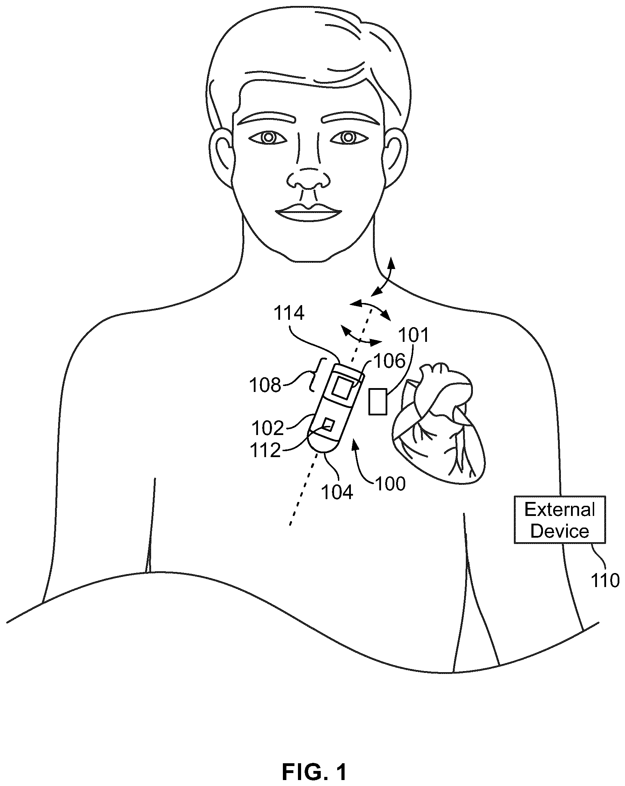

Figure 1 illustrates an example IMD 100 intended for subcutaneous implantation at a site near the heart that may include an accelerometer 112 in accordance with embodiments herein. In other embodiments, an accelerometer may be housed within a separate monitoring system (not shown) implanted within the patient and capable of communicating or outputting an accelerometer signal indicative of acceleration, posture, activity, and/or the like to the IMD 100 and/or an external device 110. The IMD 100 includes two or more spaced-apart sense electrodes 104, 106 positioned with respect to a housing 102 of the IMD 100. The sense electrodes 104, 106 provide for detection of far field electrogram signals. Header 108 includes an antenna 114 and the electrode 106. The antenna 114 is configured to wirelessly communicate with an external device 110 and/or second IMD 101 in accordance with one or more predetermined wireless protocols (e.g., Bluetooth, Bluetooth low energy, Wi-Fi, Near Field Communication, etc.). In some embodiments, conductive communication can be used to communicate between two or more of the IMD 100, IMD 101, and the external device 110. In other embodiments, a transceiver, transmitter, receiver, etc., can be used. In still further embodiments, the antenna 114 is optional. The IMD 100 can be implanted to monitor and diagnose patients experiencing issues such as but not limited to palpitations, being out of breath, idiopathic stroke, and/or fatigue.

The housing 102 includes various other components such as: sense electronics for receiving signals, including far field CA signals, from the electrodes, a microprocessor for analyzing the far field CA signals, including assessing the presence of R-waves in cardiac beats occurring while the IMD 100 is in different IMD locations relative to gravitational force, a loop memory for temporary storage of CA data, a device memory for long-term storage of CA data, the accelerometer 112 for detecting patient activity, posture, etc., and in some cases for detecting acceleration signatures indicative of heart sound, and a battery for powering components. Patient activity includes walking, jogging, running, sitting, jumping, sleeping, standing, etc.

The IMD 100 may sense far field, subcutaneous CA signals, and processes the CA signals to determine if certain conditions are detected, as discussed herein. The IMD 100 can determine heart rate (HR) based on the CA signals and determine whether the HR, alone or in combination with activity and/or posture, is indicative of an event that is a physiological condition to be monitored and/or diagnosed. If the event is indicative of a condition to be monitored and/or diagnosed, in some embodiments the CA signals can be automatically recorded in memory for subsequent transmission to the external device 110. In other embodiments, the IMD 100 may update a counter to track the number of events in a predetermined category. In some cases, the IMD 100 also can process the CA signals to detect arrhythmias and if an arrhythmia is detected, automatically record the CA signals in memory for subsequent transmission to an external device 110.

In some embodiments, the accelerometer 112 can be a chip, device, or other circuit housed within implantable medical devices (e.g. pacers, implantable cardioverter defibrillators (ICDs), ICMs, etc.) and has the capability to quantify acceleration in the three orthogonal directions (e.g., X, Y, and Z). In some cases, only one axis is used to capture activity as a binary true or false state. The accelerometer 112 further acquires information and outputs an accelerometer signal (or signals) that includes data that can be used to determine posture, such as with respect to gravity and activity. In other embodiments, the accelerometer 112 may be implantable and housed outside the IMD 100, capable of communication with the IMD 100. For example, the accelerometer 112 may include one or more structural and/or functional aspects of the device(s) described in US Published Application 2021/0350931, titled "Method and Systems for Heart Condition Detection Using an Accelerometer", filed March 8, 2021, and US patent 6,937,900, titled "AC/DC Multi-Axis Accelerometer For Determining A Patient Activity And Body Position", filed July 24, 2002.

The IMD 100 is implanted in a position and orientation such that, when the patient stands, the IMD 100 is located at a reference position and orientation with respect to a global coordinate system that is defined relative to a gravitational direction. For example, the gravitational direction may be along the Z-axis while the X-axis is between the left and right arms.

In some embodiments, the IMD 100 is capable of delivering therapy. The IMD 100 can deliver therapy to treat a heart condition that is diagnosed (e.g., identified) based at least in part on data collected by the IMD 100. In other embodiments, a second IMD 101, capable of delivering therapy, can be implanted in the patient. The second IMD can receive communications, instructions, and the like from the IMD 100 and/or the external device 110. The second IMD 101 can deliver therapy to treat a heart condition that is diagnosed (e.g., identified) based at least in part on data collected by the IMD 100.

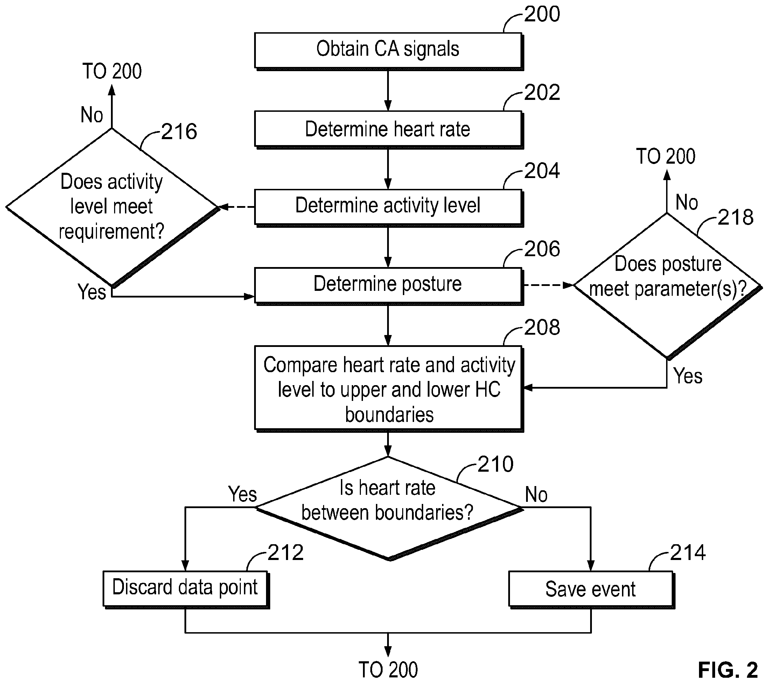

Figure 2 illustrates a method for collecting HR-AL events indicative of a physiologic condition of a patient to determine a relationship between heart rate and activity and/or posture in accordance with embodiments herein. In one example, the process of Figure 2 is performed utilizing the CA signals detected and retrieved by the systems, devices and methods described herein. The operations of Figure 2 may be implemented by hardware, firmware, circuitry and/or one or more processors housed partially and/or entirely within the IMD 100, a local external device, remote server or more generally within a health care system. Optionally, the operations of Figure 2 may be partially implemented by an IMD 100 and partially implemented by a local external device 110, remote server or more generally within a health care system. It should be recognized that while the operations of Figure 2 are described in a somewhat serial manner, one or more of the operations may be continuous and/or performed in parallel with one another. For example, the various operations of the IMD 100 may be continuous and/or performed in parallel with one another and/or other functions of the IMD 100.

At 200, one or more processors obtain a signal indicative of cardiac electrical activity (e.g., CA signals) of a heart. Such a signal can be an electrogram (EGM). For example, the signal can be an EGM obtained using an IMD and may be a measure of an intrinsic rhythm. The IMD can be the IMD 100 described herein with reference to Figures 1, 7, and/or 10 or an IMD 101 capable of therapy, such as a pacemaker, cardioverter, cardiac rhythm management device, defibrillator, neurostimulator, etc. In the following, the IMD is described with reference to the IMD 100 but the disclosure applies likewise to an IMD 101 capable of therapy.

In some embodiments, the CA signals may be continuously acquired, evaluated, and stored in memory or discarded as discussed herein. In some cases, the CA signals can be acquired for a predetermined monitoring period. A monitoring period can include the time between download sessions, wherein data collection can be continuous, intermittent, periodic, scheduled, etc. In some embodiments, the IMD 100 will acquire the CA signals for a predetermined amount of time, such as for one minute, two minutes, etc., during each hour, while in other embodiments the CA signals are acquired only during certain time periods, such as to exclude nighttime or daytime acquisition. In other embodiments, the IMD 100 will acquire a predetermined number of CA signal samples.

At 202, the one or more processors determine or generate a heart rate associated with the CA signals over a monitoring period. The monitoring period may be a relatively short period of time, such as one or more seconds, or a longer period of time, such as one or more minutes, 30 minutes, or the like. Additionally, or alternatively, the monitoring period may be defined in terms of a number of cardiac cycles (e.g., 1, 1-8, 16, 30, etc.).

At 204, the one or more processors determine or generate an activity level of the patient based on an accelerometer signal output by the accelerometer 112 over the monitoring period. The accelerometer signal is indicative of acceleration, posture, activity, etc. Accelerometer data, based on the accelerometer signal, may be generated or collected at each point in time for which a CA signal is acquired, representing a single HR-AL event or data point. Additionally, or alternatively, the accelerometer data may represent an average or other mathematical combination of accelerometer measurements over the monitoring period. For example, an HR-AL event may represent an average activity level and average heart rate over one or more seconds, one or more minutes, or the like. In some embodiments, flow moves directly from 204 to 206, and 216 is omitted. Optionally, in some cases, the accelerometer 112 may output more than one accelerometer signal, and the accelerometer data may be based on more than one accelerometer signal.

At 206, in some embodiments the one or more processors determine or generate the posture of the patient based on data collected by the accelerometer. It should be understood that 202, 204 and 206 can occur at the same point in time (or same averaging interval), acquiring activity level and/or posture associated with the heart rate at a particular point in time. In some embodiments, step 206 may be omitted.

At 208, the one or more processors compare the heart rate and activity level, for the point in time or same averaging interval, to a heart condition (HC) metric, such as to upper and lower HC boundaries within the HC metric.

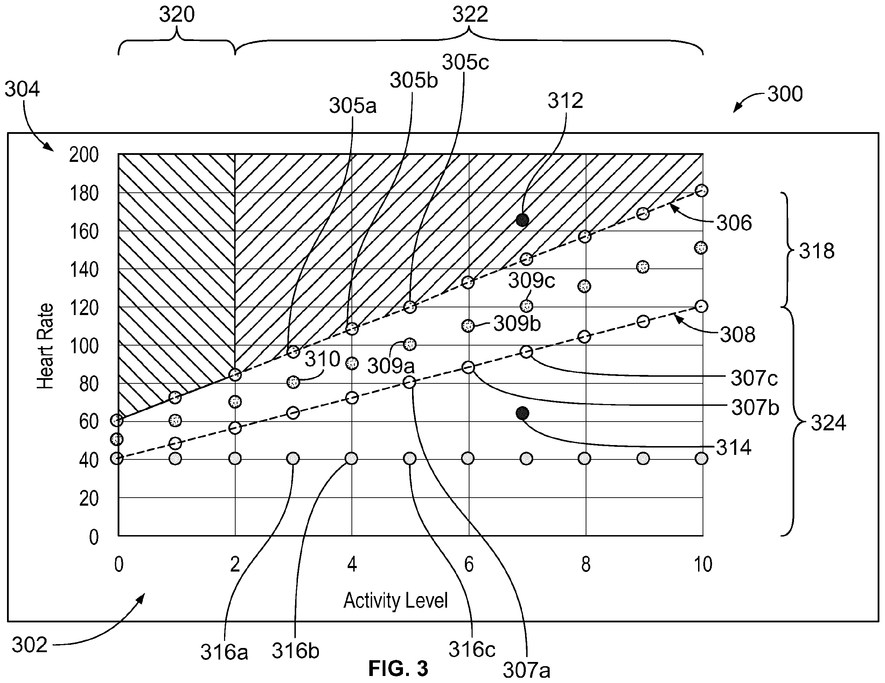

Figure 3 illustrates a graphical representation of an example of a heart condition (HC) metric 300 utilized in accordance with embodiments herein. The HC metric defines a relation between heart rates and activity levels utilized for assessing various conditions of a patient's heart in accordance with embodiments herein. Horizontal axis 302 plots activity level, while vertical axis 304 plots heart rate in beats per minute (bpm). Each data node/point may correspond to a single cardiac event HR and associated instantaneous activity level, or an average HR over a plurality of cardiac cycles and a corresponding average activity level for the same plurality of cardiac cycles.

Data nodes/points 305a, 305b, 305c (not all are individually indicated) represent separate HR-AL events and collectively form an upper HC boundary 306. Data nodes/points 307a, 307b, 307c (not all are individually indicated) represent HR-AL events and collectively form a lower HC boundary 308. Although 10 activity levels are indicated, it should be understood that the accelerometer 112 can determine more or less activity levels. In some embodiments, the activity levels can be more granular, such as to focus on a particular level of activity. Data nodes/points 309a, 309b, 309c (not all are individually indicated) represent HR-AL events detected having heart rates at activity levels that are between the upper and lower HC boundaries 306, 308. In some embodiments, the upper and lower HC boundaries 306, 308 can be developed based on populations, some of which exhibit physiologically abnormal heart conditions and some of which exhibit physiologically normal heart conditions. Additionally, or alternatively, the HC metric can be determined based at least in part on the age of the patient, physiology and/or goals of the patient, and/or determined/adjusted by a medical practitioner.

The upper HC boundary 306 and lower HC boundary 308 define relational limits correlating activity level with heart rates that are deemed physiologically normal/healthy combinations and physiologically abnormal/unhealthy combinations. Additionally, or alternatively, the upper HC boundary 306 and lower HC boundary 308 may also be considered to define relational limits correlating changes in activity level with changes in heart rate that are similarly deemed physiologically healthy combinations and physiologically abnormal/unhealthy combinations. The range/zone 318 between the upper and lower HC boundaries 306, 308 represents combinations of HRs and activity levels (HR-AL events) that correspond to physiologically normal/healthy conditions. The range/zone 320 above the upper HC boundary 306 defines combinations of HR and activity levels (HR-AL events) that correspond to one or more types of physiologically abnormal/unhealthy conditions. The range/zone 324 below the lower HC boundary 308 defines combinations of HR and activity levels (HR-AL events) that correspond to one or more types of physiologically abnormal/unhealthy conditions. In some embodiments, one or both of the upper and lower HC boundaries 306, 308 may linearly increase as HR and activity level increase. In other embodiments, one or both of the upper and lower HC boundaries 306, 308 may nonlinearly increase, may plateau, may increase at more than one rate for different activity levels, etc. However, the upper HC boundary 306 includes higher heart rates than the lower HC boundary 308 at the same activity levels such that there is a range between the thresholds wherein the HR and activity level relationship may be considered normal cardiac response.

Returning to Figure 2, at 210 the one or more processors optionally, but preferably, determine whether an HR-AL event (e.g., data point associated with the current HR and activity level) is located between the upper and lower HC boundaries 306, 308. For example, the one or more processors can determine where on the HC metric 300 the HR-AL event (data point) would be located. For example, if the data point is located at data node/point 310, such as being associated with a HR of approximately 80 bpm and activity level of 3, the data node/point 310 is between the upper and lower HC boundaries 306, 308 and is deemed to be a physiologically normal reaction of the HR to activity level. In this example, flow preferably passes to 212 and the one or more processors discard or deletes the data point and/or other data associated with the CA signals. The data node/point 310 (HR-AL event) is not saved in an HC assessment data set associated with the patient.

Optionally, a counter may maintain a count of the number of HR-AL events that are discarded at 212. For example, this information may be of interest to inform a clinician of how many or what portion of the heart beats were associated with normal activity versus the number of heart beats that were associated with abnormal activity.

Alternatively, the one or more processors may determine at 210 that the HR-AL event associated with the determined HR and activity level exceeds the upper HC boundary 306, such as data node/point 312. Alternatively, the one or more processors may determine at 210 that the HR-AL event is below the lower HC boundary 308, such as data node/point 314. When either condition is determined, at 214, the one or more processors preferably save the HR-AL event in memory, including the information associated with the data node/point 312, 314.

After the event is saved at 214 or discarded at 212 the method returns to 200.

The operations at 200 to 214 are preferably repeated over a monitoring period of time to save any/all of the HR-AL events outside of the upper and lower HC boundaries 306, 308 to build an HC assessment data set for a current monitoring period. The HC assessment data set represents a plurality of HR-AL events that correspond to data points in regions of the HC metric 300 that correspond to physiologically abnormal/unhealthy heart conditions. In some embodiments, time of occurrence and/or posture may be added to each corresponding HR-AL event. If the memory is full, older data (e.g., events) can be overwritten until the data is transmitted to the external device 110. In some embodiments, the events can be averaged over a predetermined time period, such as over a predetermined number of minutes or an hour, providing the technical advantage of saving memory space.

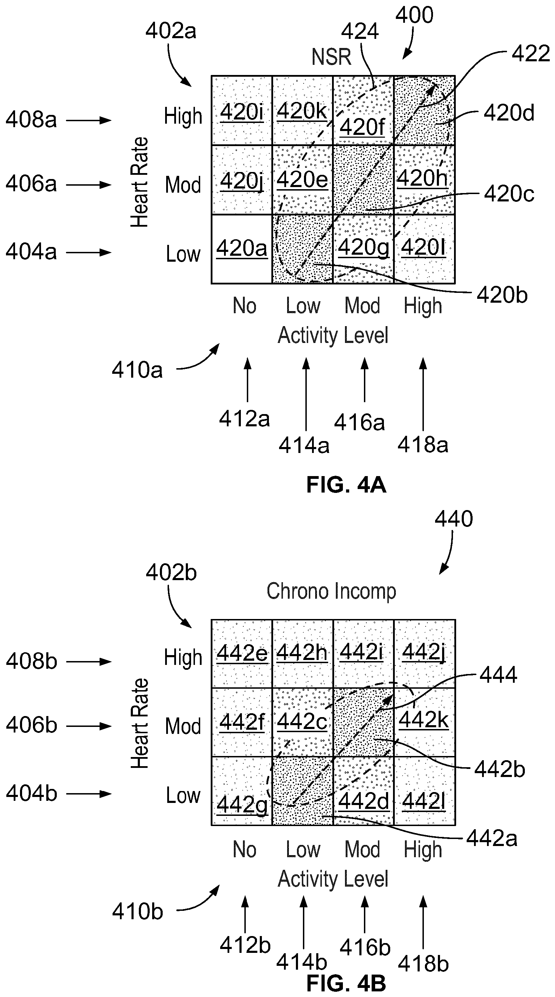

In other embodiments, the HR-AL events can be saved with less granularity, such that not every "abnormal" HR-AL event is saved at 214. Instead, a collection of counters may be maintained in the memory of the IMD 100, where each counter corresponds to a predetermined HR-AL zone. Each HR-AL zone is associated with a range of heart rates and a range of activity levels. For example, the HR-AL zones may be defined to correspond to the HR and AL blocks discussed herein in connection with Figures 4A-4F. For example, particular combinations of heart rates and activity levels can be grouped together, such as activity level zero (e.g., no activity), activity levels one through three (e.g., low activity), activity levels four through seven (e.g., moderate activity), and activity levels eight through ten (e.g., high activity), and HR can be grouped together as low HR, moderate HR, and high HR. Other smaller or larger ranges and/or additional ranges can be defined.

When an individual HR-AL event is identified, the counter for the corresponding HR-AL zone is preferably incremented in the memory of the IMD 100. For example, when an HR-AL event has a low HR and a high AL, the counter for the corresponding zone is incremented (e.g., with respect to Figure 4A, a counter for zone/block 420l would be incremented). As another example, when an HR-AL event has a low AL and a high HR, the counter for the corresponding zone is incremented (e.g., with respect to Figure 4A, a counter for zone/block 420k would be incremented. When it is desirable to transmit the values of the counters from the IMD 100 to an external device, the collection of counter values represent the HC assessment data set.

Optionally, when counters are used to track the number of HR-AL events in each zone, the IMD 100 may have sufficient memory to also store counts for the number of HR-AL events within the range between the upper and lower HC boundaries (e.g., HR-AL events associated with physiologically normal/healthy conditions). When the IMD 100 has sufficient memory to maintain counts for HR-AL zones associated with physiologically normal/healthy conditions, the decisions at 208 and 210, and the deleting operation at 212, may be omitted entirely. In this alternative implementation, flow moves from 206 (or 218 if implemented) directly to 214.

Returning to 204, in some embodiments the method passes to 216 and the one or more processors determine whether the activity level meets one or more requirements or thresholds. For example, the healthcare practitioner may only want to see events occurring at or above a minimum activity level. In some cases, this may be to assess the patient's fitness during rehabilitation, to acquire events when the patient is exerting some level of effort, and/or to save battery life in the IMD 100. In some embodiments, the healthcare practitioner may program or reprogram the IMD 100 to acquire the subset of total events after they have confirmed that the patient is not experiencing heart issues when at rest. In some cases, the healthcare practitioner can reprogram new parameters for the IMD 100 and transmit the new parameters to the external device 110 located remote from the healthcare practitioner. The next time the IMD 100 and external device 110 establish a connection session, the external device 110 can reprogram the IMD 100 and receive the data collected by the IMD 100. In further embodiments, the IMD 100 can be programmed to acquire only events below a predetermined activity level, such as an activity level of zero or one.

If the activity requirement is not met at 216, flow returns to 200. If the activity requirement is met, flow passes to 206.

In some embodiments, one or more processors can perform 216 in advance of obtaining CA signals. In this case, the IMD 100 does not sample the CA signals except when the patient is engaged at a minimum level of activity (and/or maximum level of activity). In some cases, the one or more processors may wait a period of time, such as between one minute to 10 minutes, before assessing the activity level again, further preserving battery power.

Returning to 206, in some embodiments the method passes to 218 and the one or more processors determine whether the posture meets parameters. For example, the healthcare practitioner may be interested in certain postures, such as sitting or standing, and not interested in supine or prone postures. If the posture does not meet the parameter(s), flow returns to 200. If the posture parameter is met, flow passes to 208.

The IMD 100 monitors for a request from an external device 110 to transmit the saved data. After the IMD 100 transmits the data, in some embodiments the memory can be reset, while in other embodiments, the memory will continue to function by recording new data over old data.

Returning to Figure 3, heart failure zone 320 is indicated on the HC metric 300. For example, the HR-AL events within the zone 320 have HRs exceeding the upper HC boundary 306 and activity levels from zero to approximately two. The HR-AL events in zone 320 may indicate that the patient is experiencing a heart failure condition. In other embodiments, analysis of the events within the heart failure zone 320 over time may allow the diagnosis and treatment of heart failure, the diagnosis and treatment of a worsening of a patient's condition with respect to heart failure, the monitoring of the patient's condition of heart failure over time, the analysis and treatment of the effectiveness of drugs used to treat heart failure, the analysis and treatment of the effectiveness of interventions over time, the determination and treatment of whether a patient has become resistant to medication, and the like.

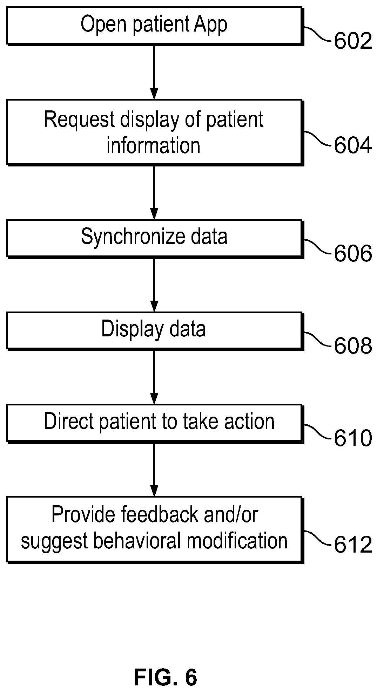

A fitness zone 322 can extend for HR exceeding the upper HC boundary 306 through approximately activity levels two through 10. For example, events within fitness zone 322 can be used to track a patient's progress during rehabilitation. In some embodiments, the patient can accomplish a test such as, but not limited to, the six-minute walk test discussed below in Figure 6, which can track a patient's fitness over time. Although not indicated, it is understood that HF zone 320 and fitness zone 322 may overlap one another. Also, zones 320, 322 may be preset, such as by the manufacturer, adjustable by the healthcare practitioner, adjustable based on patient data, symptoms, and/or diagnosis, etc. Therefore, the zones as shown in Figure 3 are examples and can include different heart rates and activity levels than those indicated.

HR-AL events that are below the lower HC boundary 308 can be used to diagnose sick sinus syndrome (SSS). With SSS, the patient's heart rate does not increase appropriately when the patient increases their activity level. For example, data nodes/points 316a, 316b, 316c (not all are indicated) extend at substantially the same HR, in this example 40 bpm, through the activity levels three through five. It is understood that there can be some variability in HR; however, the HR will remain low with respect to the activity level. In some cases, all or a subset of the data points/events that occur below the lower HC boundary 308 may indicate a diagnosis of and treatment for SSS or the need for the medical practitioner to screen the patient for SSS.

Figures 4A-4F illustrate different graphical representations for HC treatment profiles that may be formed and displayed in accordance with embodiments herein. The HC treatment profiles are formed by mapping the HC assessment data set onto an HR-AL template. The HC treatment profiles may be utilized to display the data acquired using the IMD 100 that reveals the relationship between heart rate and activity level in connection with diagnosis and treatment of a patient's heart related condition. In some embodiments, an external device 110 can process the data after the data is downloaded from the IMD 100 and display one or more graphical representation on a display, and/or email, text and/or upload graphics and/or potential diagnosis to another system. According to new and useful aspects, the HC treatment provides proactive monitoring and early diagnosis of a patient based on early disease symptoms. For example, before the patient experiences severe symptoms such as loss of consciousness, the IMD 100 and an external device 110 can be used to acquire the HR-AL events, form an HC assessment data set and display in the HC treatment profile to diagnose a patient in advance of the patient experiencing a critical or fatal event. Further, according to new and useful aspects, the graphical representations can provide ongoing monitoring, analysis and treatment to evaluate changes in a patient's condition, fitness level, and the like.

Figures 4A-4F illustrate HC treatment profiles divided into activity zones and heart rate zones, with the activity and HR zones overlapping in grid pattern. The HC treatment profiles are formed by mapping HC assessment data sets onto an HR-AL template. The HR-AL template may be presented as a grid formed by intersections of activity zones (e.g., i) no activity, ii) low activity, iii) moderate activity, and iv) high activity), and HR zones (e.g., i) low HR, ii) medium HR, and iii) high HR). Vertical axis 402 plots HR grouped into three zones (e.g., ranges or bins) and horizontal axis 410 plots activity level grouped into four zones (e.g., ranges or bins). For example, low HR range 404, moderate HR range 406, and high HR range 408 are indicated. In some embodiments, a range of HR can be established (e.g., based on the patient, age-related norms) and divided substantially equally. For example, if an entire HR range is determined to be 60 bpm to 200 bpm, each range can include approximately 46 bpm. In some embodiments, the HR range can include HR below the lower HC boundary 308. In other embodiments, the moderate HR range 406 can be larger than either of the low HR range 404 or the high HR range 408. In still further embodiments, the healthcare practitioner can establish the HR ranges 404, 406, 408. The activity level is divided into no activity 412, low activity range 414, moderate activity range 416, and high activity range 418. In some embodiments, no activity 412 can be associated with an activity level of zero, while the remaining activity levels (e.g., activity levels 1 to twelve) can be divided between the low activity range 414, moderate activity range 416 and high activity range 418. It is understood that more or fewer levels can be determined and displayed.

Figures 4A-4F present a 12-grid template based on intersections of three heart rate zones and four activity levels, although it should be understood that the template can be represented in different forms of graphical displays, such as bar charts, pie charts, etc. It is understood that larger and smaller grids can be used to tailor the display to the needs of the medical practitioner or the symptoms and/or goals of the patient. In Figure 4A, the 12 possible blocks are denoted 420a-420l. Each of blocks 420a-420l includes an "event count" or event indicator ("El indicator"), which is a graphical or numeric indication of the number of HR-AL events that occurred in the corresponding block 420a-420l. For example, the El indicator may represent a unique color or shade, a graphical pattern, a gradient, a shading density, a numeric value, and the like.

When the HC assessment data set is mapped onto the HR-AL template, a corresponding El indicator is assigned to the respective block 420a-420l. For example, a low El indicator may be assigned to blocks corresponding to a lower number of HR-AL events (e.g., zero events to a first threshold count). A lower intermediate El indicator may be assigned to blocks corresponding to a relatively intermediate number of HR-AL events (e.g., for an event count between the first threshold count and a second threshold count). An upper intermediate El indicator may be assigned to blocks corresponding to an upper intermediate number of HR-AL events (e.g., for an event count between the second threshold count and a third threshold count). A high El indicator may be assigned to blocks corresponding to a high number of HR-AL events (e.g., for an event count between the third threshold count and a fourth threshold count). Each of the EI event indicators can be displayed, for example, by filling a block with a different pattern, different color, different gradient, a flashing or otherwise "active" indication and the like based on the number of HR-AL events in the HC assessment data set.

Figure 4A illustrates a graphical representation 400 for an HC treatment profile of normal sinus rhythm (NSR) that can be measured in relatively healthy patients. As one example, the HC treatment profile of a patient with NSR may be presented as a training tool to facilitate training clinicians as to desirable and undesirable HC treatment profiles. As a further example, a HC treatment profile may be collected early in a patient's evolution of a heart condition, while the patient still exhibits NSR, to be used as a baseline relative to future HC assessment data sets collected for the same patient at later stages of a heart disease. In yet a further example, HC treatment profiles for patients with NSRs may be collected for a patient population, to be used as a baseline relative to future HC assessment data sets of patients within or outside of the patient population.

The HC assessment data set is mapped into blocks 420a-420l by assigning a corresponding El indicator to each block. For example, block 420a corresponds to HR-AL events having a HR within the low HR range 404 and no activity 412, and is assigned an El indicator associated with a lower number of HR-AL events. Blocks 420b, 420c, 420d have the greatest number of HR-AL events and are assigned the El indicator associated with a larger or maximum number of events. Blocks 420e, 420f, 420g, 420h have a higher intermediate number of HR-AL events and are assigned an El indicator for a higher intermediate number of events indicator, and blocks 420i, 420j, 420k, 420l have a lower intermediate number of HR-AL events and are assigned an El indicator for a lower intermediate number of events. The scatter plot of the HC treatment profile in Figure 4A corresponds to a patient experiencing normal sinus rhythm and would be generated if the majority of the HR-AL events occur within the range between the upper HC boundary 306 and lower HC boundary 308 (assuming HR-AL events in this range are saved).

A trend line 422 may also be calculated based on the HC assessment data set. For example, the trend line 422 can be the linear regression line from all HR-AL events in the grid. In the example of Figure 4A, for a patient experiencing NSR, the trend line 422 extends through the blocks 420b, 420c, 420d which contain the largest number of HR-AL events relative to other blocks. Patients with NSR are expected to exhibit changes in HR that are somewhat proportional to changes in activity level, namely as the HR increases the AL increases at a somewhat similar rate. Thus, the trend line 422 extends diagonally in a linear increasing manner. A healthcare practitioner can easily identify, by looking at the graphical representation 400, that the patient's HR vs. activity level is relatively normal. In some embodiments, the system can display a text diagnosis (not shown) associated with the graphical representation 400.

In some embodiments, an El indicator 424 can be used to indicate the grid locations that have a majority of the HR-AL events, while the grid locations outside the EI indicator 424 have fewer or many less HR-AL events. In other embodiments, the El indicator 424 can indicate an area within which most of the HR-AL events are expected to occur. In the example of Figure 4A, which depicts NSR, most of the expected normal HR-AL events should occur within the El indicator 424, while events occurring outside the EI indicator 424 may be considered to be abnormal. The El indicator 424 can be any shape, such as an oval, a circle, a square, "U" shaped, irregularly shaped, etc.

Figure 4B illustrates a graphical representation 440 of an HC treatment profile for a patient experiencing chronotropic incompetence. Blocks 442a, 442b have the greatest number of HR-AL events and are assigned a corresponding El indicator associated with the highest number of events. Blocks 442c, 442d include the higher intermediate number of HR-AL events and are assigned the corresponding El indicator. Blocks 442e-442l include the lower intermediate number of HR-AL events and are assigned the corresponding El indicator. In representation 440 of Figure 4B, no blocks are indicated as having the lower number of events.

A trend line 444 is calculated to extend through the blocks 442a, 442b that represent the maximum number of events. In chronotropic incompetence patients, since the HR does not increase proportionally with increasing activity, no high HR heart rate zones will be occupied by high levels of HR-AL events and the HR-AL events will have activity levels likely within low and moderate levels since patients will not be able to engage in high level activity due to the inability for their heart to increase its rate.

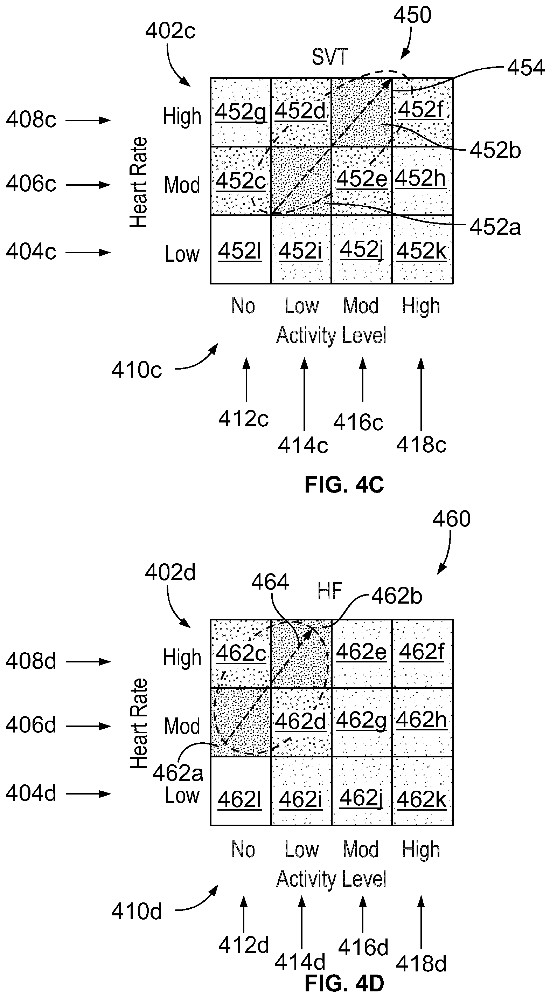

Figure 4C illustrates a graphical representation 450 of an HC treatment profile for a patient experiencing supraventricular tachycardia (SVT) (e.g., atrial fibrillation). Blocks 452a, 452b have the greatest number HR-AL events and are assigned a corresponding El indicator associated with the highest number of events. Blocks 452c, 452d, 452e, 452f include the higher intermediate number of HR-AL events and are assigned the corresponding El indicator. Blocks 452g, 452h, 452i, 452j, 452k include the lower intermediate number of HR-AL events and are assigned the corresponding indicator. Block 452l includes the lower number of HR-AL events and is assigned the corresponding El indicator.

A trend line 454 is calculated to extend through the blocks 452a, 452b that represent the maximum number of events. In SVT patients, the blocks with no or lower activity with elevated heart rate will be populated as patients undergo SVT events but will still proportionally be less than the blocks 452a, 452b.

In some embodiments, the HR-AL events may be acquired for a minimum amount of time or minimum number of events to distinguish between different diagnosis and apply and/or recommend treatment. For example, acquiring events over time can distinguish between a diagnosis of SVT and a diagnosis of heart failure.

Figure 4D illustrates a graphical representation 460 of an HC treatment profile for a patient experiencing heart failure. Blocks 462a, 462b include the greatest number of HR-AL events and are assigned the corresponding El indicator. Blocks 462c, 462d include the higher intermediate number of HR-AL events and are assigned the corresponding El indicator. Blocks 462e, 462f, 462g, 462h, 462i, 462j, 462k include the lower intermediate number of HR-AL events and are assigned the corresponding El indicator. Block 462l includes the lower number of HR-AL events and is assigned the corresponding El indicator.

A trend line 464 is calculated to extend through the blocks 462a, 462b that represent the maximum number of events. In HF patients, the profile may be close or similar to SVT patients, but the blocks with no activity and moderate HR (e.g., block 462a, 462c) will be more populated as HF patients will be inactive and their heart rates are likely to remain elevated, but, in some cases, not as high as SVT patients.

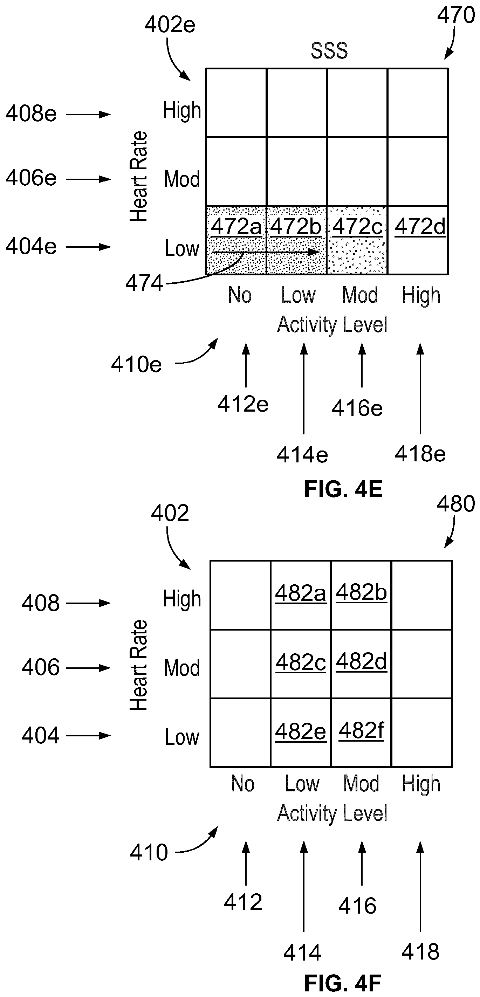

Figure 4E illustrates a graphical representation 470 for an HC treatment profile of a patient experiencing sick sinus syndrome. Blocks 472a, 472b are the most populated with HR-AL events and are assigned the corresponding El indicator. Also, a trend line 474 is calculated to extend through these blocks. Block 472c is less populated, because the patient may be less able to increase activity, given that their HR does not proportionally increase with activity. Block 472d, associated with high activity, may have few or no events as the patient is unlikely to be able to accomplish this level of activity. The remaining blocks can represent little or no events as the heart rate does not reach the moderative HR range 406e.

Figure 4F illustrates a graphical representation 480 of an HC treatment profile used to assess and track the progress of cardiac fitness. Referring again to Figure 3, the fitness zone 322 is defined as an area of interest when evaluating cardiac fitness. For example, cardiac fitness can be elicited from HR associated with light, moderate, and/or high activity levels. Applicable events can be tracked in patients that are undergoing cardiac rehabilitation. In Figure 4F, blocks 482a-482f may be of particular interest. As the number of events in blocks 482a, 482b decrease, this is an indication that the patient is able to exert low and medium level activity without having a heart rate that increases into the highest HR zone. The system can inform the patient and/or medical practitioner that the patient's fitness is improving.

Although not shown, an HC assessment profile can also be created to assess sleep, in some cases filtering the acquired event data points based on activity level and posture. Sleep can be associated with a minimum period of time when the accelerometer did not detect activity (e.g., activity level above zero), and may further be defined based on time of day. For example, with sleep apnea, the patient will stop breathing. This causes the patient's HR to drop. When the patient resumes breathing, a period of increased HR is seen. Methods and apparatus to detect HR, activity, and posture described herein can be used together with methods and apparatus discussed in US Patent Application 2021/0369191 titled "Methods, Systems, and Devices for Detecting Sleep and Apnea Events", filed April 20, 2021.

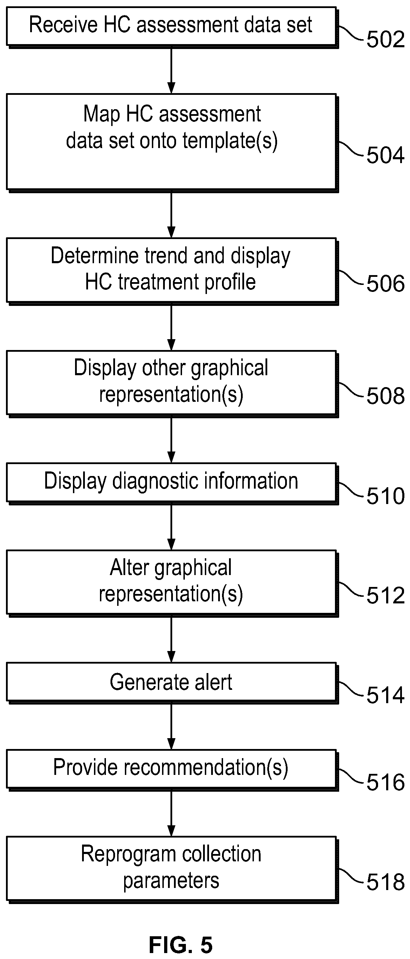

Figure 5 illustrates a method for monitoring a physiologic condition, determining a treatment and delivering the treatment in accordance with embodiments herein. In one example, the process of Figure 5 is performed utilizing the HC assessment data sets collected by the IMD 100 as described above. The operations of Figure 5 may be implemented by hardware, firmware, circuitry and/or one or more processors housed partially and/or entirely within the IMD 100, a local external device 110, remote server or more generally within a health care system. Optionally, the operations of Figure 5 may be partially implemented by an IMD 100 and partially implemented by a local external device 110, remote server or more generally within a health care system. It should be recognized that while the operations of Figure 5 are described in a somewhat serial manner, one or more of the operations may be continuous and/or performed in parallel with one another. For example, the various operations of the IMD 100 and system may be continuous and/or performed in parallel with one another and/or other functions of the IMD 100 and system. The method as shown in Figure 5 may, alternatively, be implemented involving an IMD 101 capable of providing therapy.

At 502, the one or more processors receive an HC assessment data set for a select patient. For example, a smart device, home monitoring device, clinician programmer or other external device 110 may establish a wireless connection with an IMD 100 and receive the HC assessment data set from the IMD 100. Additionally, or alternatively, the smart device, home monitoring device, clinician programmer or other external device 110 may obtain the HC assessment data set by reading local memory or accessing a patient care network. Optionally, a healthcare provider can utilize an external device 110, such as computer or smart device to access a patient care network, such as the Merlin.net.^{™} patient care network operated by Abbott Laboratories (headquartered in the Abbott Park Business Center in Lake Bluff, III.) to obtain the HC assessment data directly from an IMD 100, from a patient medical record or other memory.

At 504, the one or more processors map the HC assessment data set onto one or more corresponding templates to build an HC treatment profile. For example, one or more processors may utilize a template having a grid pattern divided into the HR zones and AL zones as discussed above in connection with Figures 4A-4F. The one or more processors step through the HC assessment data set and determine a count of HR-AL events in each zone or block. For purposes of illustration only, assume that the HC assessment data set exhibited a distribution of HR-AL events similar to Figure 4B.

Accordingly, the one or more processors determine that blocks 442a, 442b have the greatest number of HR-AL events and the one or more processors assign a "high count" El indicator (e.g., red) to these blocks. The one or more processors determine that blocks 442c, 442d have a higher intermediate number of HR-AL events and assign a second intermediate El indicator (e.g., blue) to these blocks. The one or more processors determine that blocks 442e-442l include the lower intermediate number of HR-AL events and assign a first intermediate El indicator (e.g., green) to these blocks. The one or more processors determine that no HR-AL events are in the blocks associated with the lower number of HR-AL events.

At 506, the one or more processors then display the HC treatment profile. Optionally, at 506, one or more processors may also calculate or generate a trend line 444 which extends through blocks 442a, 442b. As explained herein, the trend line 444 extends through the blocks containing the majority of the HR-AL events. The trend line 444 may be displayed with the HC treatment profile. Optionally, the trend line 444 may not be calculated. The HC treatment profile is readily recognized as indicative of chronotropic incompetence, as the HR does not increase proportionally with increasing activity, and no high HR zones will be occupied by HR-AL events. The HR-AL events will have activity levels likely within low and moderate levels since patients will not be able to engage in high level activity due to the inability for their heart to increase its rate.

Optionally, at 506 the one or more processors may determine and display a diagnosis and treatment associated with the HC treatment profile. For example, the one or more processors may determine that the patient is experiencing normal sinus rhythm, heart failure, sick sinus syndrome, chronotropic incompetence, SVT, increased fitness, decreased fitness, etc.

At 508, the one or more processors may display one or more other graphical representations. For example, the one or more processors can identify templates, such as age-adjusted templates, of similar patients for comparison display. For example, a reference template may be displayed in combination with the HC treatment profile. The reference template may represent at least one of: i) an age-adjusted template, ii) a template associated with one of chronotropic incompetence, sick sinus syndrome, heart failure, fitness level of the patient, or fitness level of a population, or iii) a graphical representation based on a second HC assessment data set collected over a second monitoring period for the patient. In another example, previous data from the instant patient can be displayed for comparison. The display of multiple representations from the same patient over time can show a comparison of the disease progression, improvement, or lack of change, of the patient. In some embodiments, the one or more processors can display templates and graphical representations based on input from the healthcare practitioner or presets that have previously been defined.

At 510, the one or more processors may determine and display diagnostic information relating the various graphical representations. For example, the one or more processors can display an age-related template associated with a particular heart status, such as normal sinus rhythm, heart failure, chronotropic incompetence, SSS, SVT, etc., to confirm that the diagnosis is correct. In other embodiments, the one or more processors can provide diagnostic information to determine changes in the patient, such as no change or similar within predetermined limits, improving, deteriorating, etc., in comparison with previous data acquired from the instant patient. Percentage improvement/deterioration may be displayed. In some embodiments, graphical representations may be overlayed and displayed, such as using a variety of colors and/or symbols, to emphasize the similarity or differences between images. In some embodiments, a probability that the patient has a particular diagnosis can be determined and displayed.

At 512, in response to user input, the one or more processors can alter the graphical representation. For example, the user may wish to see HC assessment data associated with a subset of postures, expand the display of data associated with particular heart rate ranges and/or activity levels, etc. In other embodiments, the user may wish to see HC assessment data associated with a time of day, such as when the patient is sleeping or awake.

In some embodiments, body posture information, such as the posture determined at 206 of Figure 2, can be used to stratify the events in the scatter plot(s) of Figures 4A-4E to improve precision. For example, the medical practitioner may want to only see the HR-AL events associated with sitting, standing, walking, etc., while not including events detected when the patient is prone or supine. This can, for example, eliminate data collected while the patient is sleeping or resting.

At 514, in some embodiments the one or more processors can generate an alert, if warranted. For example, if the one or more processors determine that the patient's condition requires immediate attention, or if the patient's condition has deteriorated beyond predetermined thresholds with respect to standards or previous patient data, an alert can be generated. The alert can be displayed on the display and/or transmitted via text, email, phone, etc., to one or more medical caregivers.

In some embodiments, the one or more processors can generate the alert without displaying any images. For example, the one or more processors can process and evaluate the HC assessment data set after the data set is transmitted from the IMD 100. This provides the advantage of alerting medical practitioners immediately before the healthcare professional has an opportunity to review the data. In some cases, healthcare practitioners can set thresholds to determine when alerts are sent and what alerts are sent. This provides the advantage of minimizing the amount of intermediate data healthcare practitioners have to personally review, while ensuring that a patient's health is protected and problems are identified in an timely manner.

At 516, in some embodiments the one or more processors can provide recommendation(s) for caring for the patient. The recommendation may represent a treatment to be delivered. For example, the one or more processors may access information on the patient care network. If the patient is taking medication, the one or more processors may recommend evaluating either the dose or medication, and may recommend changing one or more of a pharmaceutical agent, dosage or dosing regimen. If the patient is deteriorating beyond predetermined limits over time, the one or more processors may recommend adding medication, implanting an IMD 101 capable of providing therapy, programming or reprogramming an implanted IMD 101 capable of providing therapy to adjust a current therapy, and the like. Recommendations can be made for the patient to increase or decrease activity, such as to approve more strenuous physical exertion. In some embodiments, the one or more processors can alert the healthcare practitioner to trend(s) seen in the patient data.

In other embodiments, if the data indicates that the patient is in heart failure, such as shown in Figure 4D, the one or more processors can indicate the diagnosis of heart failure and suggest next steps, such as additional tests (e.g., imaging such as echocardiography, treadmill stress test, blood test) to confirm the diagnosis, such as evaluation of heart rate variability, heart sounds, impedance, ejection fraction, stroke volume, evaluation for hypertension, etc.

At 518, the one or more processors can adjust collection parameters of the patient data. For example, the healthcare practitioner may determine that more or less data should be acquired. If the patient has exhibited no negative events during sleep, the parameters may be adjusted to not collect data during a defined time window or when the activity level is zero. This may save battery power, extending the life of the IMD 100. The healthcare practitioner may wish to adjust the upper HC boundary 306 and/or lower HC boundary 308. New collection parameters can be sent to the external device 110 for downloading and reprograming the IMD 100 the next time the IMD 100 connects to transmit data to the external device 110.

Referring again to Figure 4D, the user may wish to filter the data to show what postures are associated with the high or moderate heart rate and no or low activity. For example, the healthcare practitioner can select to display only data when the patient has no activity and is prone or supine. This can in some cases be a proxy for sleep. If many events are displayed, the patient may be evaluated for a sleep disorder such as apnea.

In accordance with new and unique aspects herein, the patient's health can be monitored and tracked in a systematic manner, generating and providing qualitative data and diagnostic information to diagnose a patient's condition.

In some embodiments, an IMD 101 can be configured to deliver therapy to treat the physiologically abnormal heart condition designated or identified by the HC assessment data set. For example, the IMD 101 that collects the HR-AL events can be an implantable device that delivers therapy, such as pacing, shocking, etc., to treat the physiologically abnormal heart condition designated by the HC assessment data set. In other examples, a second IMD 101 implanted within the patient can deliver therapy to treat the physiologically abnormal heart condition designated by the HC assessment data set. The second IMD 101 can receive programming parameters via the IMD 100 that collects the HR-AL events, the external device 110, a programmer, etc., and implement the programming parameters. In other embodiments, the IMD 100 and/or second IMD 101 can adjust therapy based on the HC assessment data set.

Figure 6 illustrates a method for interfacing with a patient application in accordance with embodiments herein. In accordance with new and unique aspects herein, a patient application can provide benefits by displaying real-time or near-real-time information to the patient without having to wait for a healthcare appointment or communication with a healthcare practitioner. The patient application can also provide suggestions that are tailored to the specific patient's condition and fitness level.

In one example, the process of Figure 6 is performed utilizing the events, CA signals, activity information, and/or posture information detected and retrieved by the systems and methods described herein. The operations of Figure 6 may be implemented by hardware, firmware, circuitry and/or one or more processors housed partially and/or entirely within the IMD 100, the IMD 101, a local external device 110, remote server or more generally within a health care system. Optionally, the operations of Figure 6 may be partially implemented by an IMD 100 and/or IMD 101 and partially implemented by a local external device 110, remote server or more generally within a health care system. It should be recognized that while the operations of Figure 6 are described in a somewhat serial manner, one or more of the operations may be continuous and/or performed in parallel with one another. For example, the various operations of the IMD 100 may be continuous and/or performed in parallel with one another and/or other functions of the IMD 100.

Although the method is written primarily from the viewpoint of a patient's personal external device, it should be understood that other devices can include the patient application and that processes can be spread across multiple devices.

At 602, one or more processors can open a patient application (App) on an external device, such as in response to an input via a keyboard, voice command, GUI, etc. In some embodiments, the external device can be the same external device 110 that communicates with the IMD 100, in which case the patient App can be downloaded onto the external device 110 or included with program instructions provided on the external device 110. In other embodiments, the external device can be a patient's personal device such as a smartphone or tablet, having the patient App downloaded and capable of communication with the external device 110.

At 604, in response to patient input via the patient App, the one or more processors request various display(s) of patient information that have been collected by the IMD 100. In some cases, the patient App can be configured by the healthcare practitioner and/or patient to display a subset of data. The patient App can configure the data such as to display data acquired over a desired date / time range, acquired during particular time periods (e.g., nighttime, daytime, during sleep), display data associated with certain postures and/or activity levels, and the like. In some embodiments, a patient may be restricted to one or two views conveying the information, while in other embodiments the patient may be given a higher level of access to create and display different customized views.

At 606, in some embodiments, the one or more processors request a synchronization process between the user's device and the external device 110. For example, the patient App may request the data needed to support display of the requested patient information. In some embodiments, wherein the patient is interacting with the Patient App on the external device 110, there is no need to transmit/transfer data. In some embodiments, if the patient has not accomplished the transfer process for transmitting data from the IMD 100 to the external device 110 within a predetermined time (e.g., a day, a week, an hour, thirty minutes), the patient App may direct the patient to complete the transfer process.

At 608, in response to receiving / having current data, the one or more processors provide the information to the patient, such as on a display, touchscreen, external monitor, printer, etc. For example, text and various graphic relationships in text, bar graph, pie chart, etc., can be presented. The data can be tracked over time to show improvement in heart condition and/or deterioration of heart condition as disease progresses, such as weekly, monthly, etc.

By way of example, if a patient is working to improve their fitness during cardiac rehabilitation, the percentage of time the patient's HR exceeded the upper HC boundary 306 can be displayed. This metric can be displayed over days, weeks and/or months, showing the progress of the patient. In some embodiments, a metric reflecting progress accomplished each month can be provided. This information can be accompanied by comments praising the patient for their efforts, encouraging the patient to continue to exercise, request the patient to increase their level and/or duration of exercise, suggest particular workouts, etc.

In some embodiments, the patient can granularly select to view data during certain activities, such as walking, running, climbing stairs, at selected activity levels, etc. The patient App can display this data over time (e.g., weekly, monthly, etc.), showing progress of the patient, such as by highlighting a decreased HR during certain activities over time.

At 610, the one or more processors can direct, such as through a message on the display of the external device 110, for the patient to take a particular action. For example, the patient may be directed to do a six-minute walk test, as described in US patent application 2021/0350931 titled "Method and Systems for Heart Condition Detection Using an Accelerometer", filed March 8, 2021, and US Patent 7,177,684 titled "Activity monitor and six-minute walk test for depression and CHF patients", issued February 13, 2007. Results of multiple six-minute walk tests can be compared and displayed to the patient over time to show change or maintenance of fitness level. In other embodiments, the patient can be directed to complete a certain workout, a certain number of minutes at a particular activity level, and the like. This data can be tracked and changes displayed over time (e.g., weekly, monthly, etc.) to show a patient's progress. A six-minute walk test is merely an illustrative, but non-limiting, example of the particular action to be taken by the patient at 610. Also other examples of activity tests could be used, such as, cardiopulmonary exercise testing (CPET), treadmill or cycling tests, etc.

At 612, the one or more processors can provide feedback and/or suggest behavioral modifications. For example, in some embodiments the patient App can run in the background on the patient's device. The patient App can provide reminders, such as through a pop-up, a text, an email, a phone or smartwatch alert, etc., to take an action, suggest behavioral modification, provide information on predetermined metrics, and the like. For example, the patient App can inform the patient that their activity goals have not been met, such as a minimum number of minutes above a predetermined activity level. In accordance with new and unique aspects herein, the minutes above a predetermined activity level can be accurately tracked as the IMD 100 is continuously monitoring the patient. In contrast, an external device 110, such as a wearable relies on the battery being powered, and the device being properly worn by the patient to acquire data, which may not accurately reflect patient activity as a wearable is not positioned within the torso of the patient.

In other embodiments, the patient App can remind the patient to take medication. In still further embodiments, the patient App can ask the patient to confirm that they have taken their medication, such as in response to the patient's data indicating an undesirable change. If the patient confirms that they have taken their medication, the patient App can instruct the patient to contact their provider and provide a level of importance, such as immediately seek help, contact today, discuss with your provider at your next appointment, and the like. Similarly, the patient App can forward patient responses to the healthcare network, and in some cases send an alert to healthcare practitioners.

In still further embodiments, the patient App can evaluate the patient's sleep and provide a metric based on the patient's sleep quality, such as by reviewing data acquired during a predetermined time window and/or when the patient's activity level is zero. Sleep quality can be tracked over time. Heart rate variability, maximum HR, and/or minimum HR can be determined and displayed, showing changes over time. Suggestions to improve sleep quality can be displayed interactively to the patient. Sleep can be further evaluated using the method, systems and techniques discussed in US Patent Application 2021/0369191 titled "Methods, Systems, and Devices for Detecting Sleep and Apnea Events", filed April 20, 2021.

In yet further embodiments, the patient App can be a communication tool to provide information to and ask questions of healthcare practitioners. Healthcare practitioners can ask and answer questions, provide information, and respond to patient concerns.

In some embodiments, the patient App can provide data associated with the HR, activity level, and posture acquired by the IMD 100, while additionally tracking medication and providing reminders for taking medication, tracking activity, providing quality information regarding a patient's sleep and fitness level, and providing a vehicle for sending messages to and receiving messages from healthcare practitioners. In accordance with new and unique aspects herein, the patient App manages the care of the patient and provides extensive personalized information to the patient without the patient having to access multiple different Apps and/or systems.

In some embodiments, an IMD 101 capable of providing therapy to a patient, such as but not limited to a pacemaker, cardiac resynchronization therapy (CRT) device, subcutaneous implantable cardioverter defibrillators (SICD) device, and the like can implement the methods, aspects and embodiments described herein. When a diagnosis is determined or generated, such as sick sinus syndrome or heart failure, the therapy device can implement therapeutic routines (e.g., pacing) to actively treat the patient.

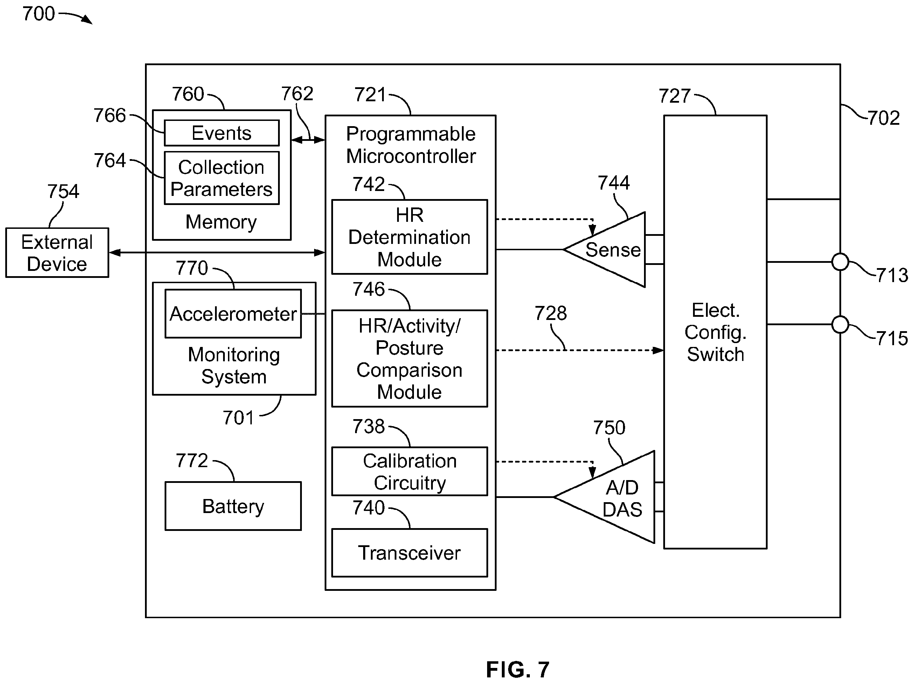

Figure 7 shows a block diagram of the IMD 700 formed in accordance with embodiments herein. The IMD 700 in Figure 7 could be the IMD 100 in Figure 1 or the IMD 101 in Figure 1. The IMD 700 has a housing 702 to hold the electronic/computing components. The housing 702 (which is often referred to as the "can," "case," "encasing," or "case electrode") may be programmably selected to act as an electrode for certain sensing modes. Housing 702 further includes a connector (not shown) with at least one terminal 713 and optionally additional terminals 715. The terminals 713, 715 may be coupled to sensing electrodes that are provided upon or immediately adjacent the housing 702. Optionally, more than two terminals 713, 715 may be provided in order to support more than two sensing electrodes, such as for a bipolar sensing scheme that uses the housing 702 as a reference electrode. Additionally, or alternatively, the terminals 713, 715 may be connected to one or more leads having one or more electrodes provided thereon, where the electrodes are located in various locations about the heart. The type and location of each electrode may vary.

A switch 727 is optionally provided to allow selection of different electrode configurations under the control of microcontroller 721. The electrode configuration switch 727 may include multiple switches for connecting the desired electrodes to the appropriate I/O circuits, thereby facilitating electrode programmability. The switch 727 is controlled by a control signal 728 from the microcontroller 721. Optionally, the switch 727 may be omitted and the I/O circuits directly connected via terminals 713, 715.

The IMD 700 includes sensing circuitry 744 selectively coupled to one or more electrodes that perform sensing operations, through the switch 727 to detect cardiac activity data indicative of cardiac activity. Optionally, the sensing circuitry 744 may be removed entirely, and the microcontroller 721 performs the operations described herein based upon the CA signals from the A/D data acquisition system 750 directly coupled to the electrodes. The output of the sensing circuitry 744 is connected to the microcontroller 721 which, in turn, determines when to store the cardiac activity data of CA signals (digitized by the A/D data acquisition system 750) in memory 760.

The IMD 700 includes the programmable microcontroller 721 that controls various operations of the IMD 700, including cardiac monitoring. Microcontroller 721 includes a microprocessor (or equivalent control circuitry), RAM and/or ROM memory, logic and timing circuitry, state machine circuitry, and I/O circuitry. The microcontroller 721 is configured to implement the operations described herein in connection with obtaining and analyzing accelerometer signals, such as to generate accelerometer data.

The microcontroller 721 may also include optional calibration circuitry 738 that is configured to implement calibration operations of accelerometer 770. Among other things, the calibration circuitry 738 obtains baseline accelerometer signals from the accelerometer 770 in connection with specific patient postures. The postures may include supine, standing, laying on a right side, laying on a left side, angled, or the like. The calibration circuitry 738 may also calculate synthetic baseline accelerometer signals based on orthogonal baseline accelerometer signals that are directly measured by the accelerometer 770. Although not shown, the microcontroller 721 may further include other dedicated circuitry and/or firmware/software components that assist in monitoring various conditions of the patient's heart and managing pacing therapies.

In some cases, the IMD 700 is further equipped with a transceiver 740 or other communication technology, such as a transmitter, receiver, communication modem (modulator/demodulator), etc., to enable wireless communication. In one implementation, the communication transceiver 740 uses high frequency modulation, for example using RF, Bluetooth, or Bluetooth Low Energy telemetry protocols. The signals are transmitted in a high frequency range and will travel through the body tissue in fluids without stimulating the heart or being felt by the patient. The communication transceiver 740 may be implemented in hardware as part of the microcontroller 721, or as software/firmware instructions programmed into and executed by the microcontroller 721. Alternatively, the transceiver 740 may reside separately from the microcontroller 721 as a standalone component. The transceiver 740 facilitates data retrieval from a remote monitoring network. The transceiver 740 enables timely and accurate data transfer/transmission directly from the patient to an electronic device utilized by a physician. In other cases, communication between the IMD 700, 100, the external device 754, 110, and/or IMD 101 can be accomplished using conductive communication.

By way of example, the external device 754, which may the external device 110 in Figure 1, may represent a bedside monitor installed in a patient's home and utilized to communicate with the IMD 700 while the patient is at home, in bed or asleep. The external device 754 may be a programmer used in the clinic to interrogate the IMD 700, retrieve data and program detection criteria and other features. The external device 754 may be a patient data entry (PDE) device (e.g., smartphone, tablet device, laptop computer, smartwatch, and the like) that can be coupled over a network (e.g., the Internet) to a remote monitoring service, medical network and the like. The external device 754 facilitates access by physicians to patient data as well as permitting the physician to review real-time accelerometer data as obtained by the IMD 700.

The microcontroller 721 is coupled to the memory 760 by a suitable data/address bus 762. The memory 760 stores the accelerometer signals, accelerometer data, reference posture data sets, cardiac activity signals, as well as the markers and other data content associated with detection and determination of the condition of the heart of the patient.

In some embodiments, the accelerometer 770 may be part of a monitoring system 701, or may represent accelerometer circuits or one or more accelerometers, such as a three-dimensional (3D) accelerometer. The accelerometer 770 may utilize, for example, piezoelectric, piezoresistive, and/or capacitive components, to convert the mechanical motion of the 3D accelerometer into an electrical signal received by the microcontroller 721. By way of example, the 3-D accelerometer may have three outputs/channels that generate three corresponding electrical signals indicative of motion in three corresponding directions, namely X, Y and Z directions. The electrical signals associated with each of the three directional components may be divided into different frequency components to obtain different types of information therefrom.

The accelerometer 770 obtains device location information with respect to gravitational force while the IMD 700 obtains cardiac activity signals in connection with multiple cardiac beats. In one example, the accelerometer 770 includes the accelerometer as described in relation to Figure 1. The microcontroller 721 may utilize the accelerometer signals from the accelerometer 770. While shown as being included within the housing 702, the accelerometer 770 may be external to the housing 702, yet still, be implanted within or carried by the patient.

A battery 772 provides operating power to all of the components in the IMD 700. The battery 772 is capable of operating at low current drains for long periods of time. The battery 772 also desirably has a predictable discharge characteristic so that elective replacement time can be detected.

The IMD 100 can be used to monitor a physiologic condition of a patient. An HR determination module 742 can receive data from the sensing circuitry 744 and determine an HR associated with the signals (e.g., CA signals) over a monitoring period that includes a plurality of cardiac cycles. Over the monitoring period, the accelerometer 770 can output an accelerometer signal indicative of acceleration, an activity level and/or posture associated with the HR. An HR/activity/posture comparison module 746 can identify events occurring at points in time within the monitoring period that have HR and associated activity level and/or posture that adhere to collection parameters. For example, collection parameters 764 can be stored in the memory 760 and include information such as upper HC boundary 306 and lower HC boundary 308, wherein the upper and lower HC boundaries 306 and 308 increase as the activity level increases, and the upper HC boundary includes higher heart rates than the lower HC boundary at the same activity levels. In response to identifying events wherein the determined HR i) exceeds the upper HC boundary 306 at the determined activity level or ii) is below the lower HC boundary 308 at the determined activity level, events 766, such as HR-AL events and one or more HC assessment data set, can be stored in the memory 760. In some embodiments, a display, such as a display associated with an external device 754, phone, computer, tablet, etc., displays a graphical relationship representing the HR and the activity level and/or posture of at least a portion of the events in connection with monitoring or diagnosing the physiological condition of the patient.

In some embodiments, the IMD 700 may also include a pulse generator (not shown) connected to the electrodes via the terminals 713, 715 and optionally through the switch 727. In such an embodiment, the microcontroller 721 is connected to the pulse generator and configured to control the generation of electrical pulses, such as pacing pulses, by the pulse generator, which are applied to a tissue in the patient, typically the patient's heart, via the electrodes.

In some embodiments, a system for monitoring a physiologic condition of a patient, such as a heart condition, includes the IMD 700 configured to be implanted in the patient. The accelerometer 770 in the IMD 700 is configured to output an accelerometer signal; and sensing circuitry 744 is configured to sense a cardiac activity (CA) signal over a plurality of cardiac cycles. A memory 760 is configured to store program instructions and one or more processors, such as the microcontroller 721 that is coupled to the accelerometer 770 and the sensing circuitry 744, execute the program instructions. The HR determination module 742 determines a heart rate (HR) at a point in time based on the CA signal. The one or more processors determine an activity level (AL) at the point in time based on accelerometer data that is based on the accelerometer signal output by the accelerometer 770, the HR and AL forming an HR-AL event. The HR/Activity/Posture comparison module 746 compares the HR-AL event to a heart condition (HC) metric that defines combinations of HRs and ALs representing a physiologically normal heart condition and a physiologically abnormal heart condition, and identifies a physiologically abnormal heart condition based on the comparison.

In other embodiments, the one or more processors 721 repeat the determining and comparing operations to build an HC assessment data set, and save the HC assessment data set in the memory 760 of the IMD 700. The one or more processors can transmit the HC assessment data set to the external device 754 or a second IMD configured to be implanted in the patient (e.g., IMD 101), and the HC assessment data set can be utilized to identify the physiologically abnormal heart condition.

In still further embodiments, a second IMD, such as IMD 101 of Figure 1, can be implanted and deliver therapy to treat the physiologically abnormal heart condition identified by the HC assessment data set.

In some embodiments, the IMD 700 can include the pulse generator configured to generate electrical pulses to be applied to the patient's heart, and the one or more processors are configured to control the pulse generator to generate pacing pulses to treat the identified physiologically abnormal heart condition.

In some cases, an external device, such as the external device 754, can receive the HC assessment data set and map the HC assessment data set onto an HR-AL template to form a HC treatment profile. Optionally, a display of the external device can display a graphical representation of the HC treatment profile. In other cases, the HC treatment profile is divided into activity zones for i) no activity, ii) low activity, iii) moderate activity, and iv) high activity, and further divided into HR zones for i) low HR, ii) medium HR, and iii) high HR, the HC assessment data set mapped into the activity and HR zones.

In some cases, the HC treatment profile can indicate an increase in a number of the HR-AL events at a relatively higher HR over at least two activity levels.

Optionally, the display can also display a reference template in combination with the HC treatment profile, the reference template representing at least one of: i) an age-adjusted template, ii) a template associated with one of chronotropic incompetence, sick sinus syndrome, heart failure, fitness level of the patient, or fitness level of a population, or iii) a graphical representation based on a second HC assessment data set collected over a second monitoring period for the patient.

In some embodiments, the HC metric includes upper and lower HC boundaries 306, 308, wherein a range between the upper and lower HC boundaries 306, 308 corresponds to physiologically healthy HR-AL events, and the regions above the upper HC boundary 306 and below the lower HC boundary 308 correspond to physiologically unhealthy HR-AL events.

In other embodiments, the one or more processors delete the HR-AL events that fall within a range of the HC metric corresponding to the combinations of HRs and ALs representing the physiologically normal heart condition such that the HC assessment data set excludes the HR-AL events that fall within the range.

In some cases, the one or more processors to save in the memory 760, in the HC assessment data set, the HR-AL events that fall in regions of the HC metric corresponding to the combinations of HRs and ALs representing the physiologically abnormal heart condition.

In still further embodiments, in response to the physiologically abnormal heart condition being identified, the HC assessment data set prescribes treatment for at least one of i) chronotropic incompetence, ii) sick sinus syndrome, iii) heart failure, iv) sleep apnea, or v) fitness level of the patient. In some embodiments, the HC assessment data set prescribes no treatment when the HC assessment data set is indicative of the physiologically normal heart condition.

In some cases, the one or more processors can determine, using the accelerometer data based on the accelerometer signal output by the accelerometer 770, a posture from within a plurality of postures over the monitoring period, and wherein each of the HR-AL events saved in the HC assessment data set in the memory 760 is associated with the corresponding posture at the corresponding point in time. In other cases, the HC assessment data set excludes at least a portion of the HR-AL events associated with at least one of the postures.

In further embodiments, the one or more processors transmit the HR-AL event or a result of the comparison to a second device.

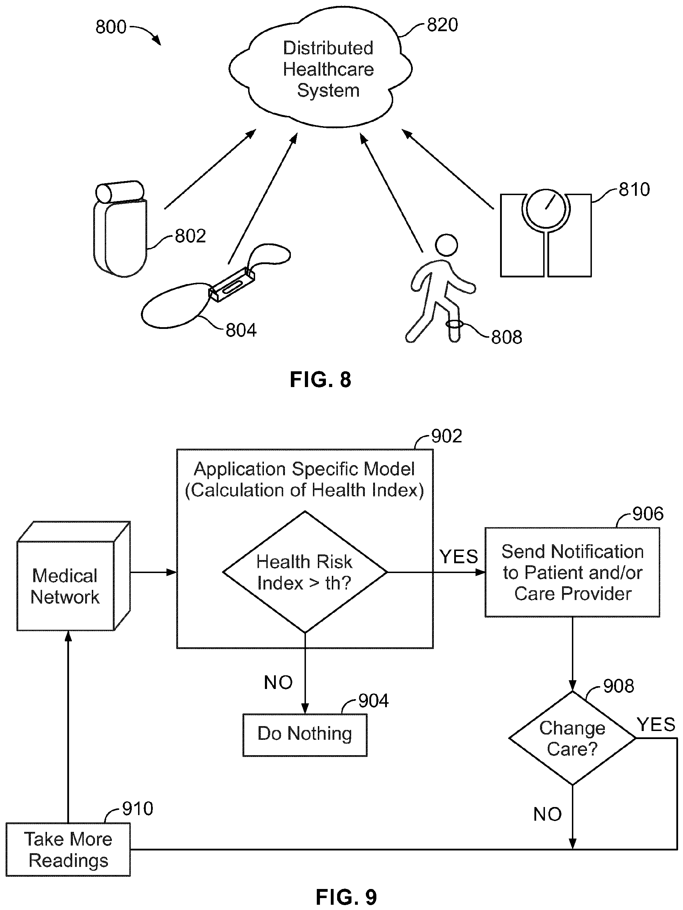

Figure 8 illustrates a digital healthcare system 800 implemented in accordance with embodiments herein. The system integrates heart rate information, accelerometer signals and the other information derived from accelerometer signals with other health data in connection with monitoring a patient condition, progression of a health condition, trends in a patient's health condition, treatment, changes in therapy/medication and the like. The healthcare systems may include wearable PDE devices that communicate with an IMD and/or accelerometer and a remote database and/or other external device. As a result, the healthcare system may monitor health parameters of a patient, including HR and accelerometer data, and provide a treatment diagnosis for the patient based on the monitored health parameters.

The system may be implemented with various architectures, that are collectively referred to as a healthcare system 820. By way of example, the healthcare system 820 may be implemented as described herein. The healthcare system 820 is configured to receive data from a variety of external and implantable sources including, but not limited to, active IMDs 802 (e.g., IMD 101) capable of delivering therapy to a patient, passive IMDs 804 (e.g., ICM, IMD 100) or sensors, wearable sensors 808, and point-of-care (POC) devices 810 (e.g., at home or at a medical facility). Any of the IMD 802, IMD 804, and/or sensor 808 may implement an accelerometer circuity and perform the analysis of accelerometer signals as described herein. The data from one or more of the external and/or implantable sources is obtained and transmitted to one or more secure databases within the healthcare system 820. Optionally, the patient and/or other users may utilize a PDE device, such as a smart phone, tablet device, etc., to enter data. For example, a patient may use a smart phone to provide feedback concerning activities performed by the patient, a patient diet, nutritional supplements and/or medications taken by the patient, how a patient is feeling (e.g., tired, dizzy, weak, good), etc.

Figure 9 illustrates a high-level flowchart of a method, implemented by the medical network of Figure 8, for processing current or real-time HR data, accelerometer data and/or IMD data in accordance with embodiments herein. The healthcare system includes one or more computing devices (e.g., servers, local external devices, MP devices) that are configured to obtain and process HR data, accelerometer data and/or IMD data. Upon receipt of new (or changes in) HR data, accelerometer data and/or IMD data, at 902, the processor of the computing device(s) identifies one or more application specific models or ASM to analyze the HR data, accelerometer and/or IMD data. Optionally, the analysis by the ASM may incorporate the additional HR data, accelerometer data and/or IMD data into any relevant trend tracked in connection with the present patient. The application specific model may be implemented in various manners, as described herein, including but not limited to lookup tables, decision trees, machine learning algorithms and the like. At 902, the processor calculates a risk weighted composite index based on the incoming HR, accelerometer data and/or IMD data, alone or in combination with previously stored HR data, accelerometer and IMD data. For example, a patient may perform, for instance, a six-minute walk test and the system may determine that the current six-minute walk test took 20% longer than a prior walk test or an average for multiple prior walk tests. The increase in time to perform the walk test may represent a risk weighted composite index increase. At 902, the processor also generates a treatment diagnosis based on the HR data, IMD data and accelerometer data. For example, the processor may adjust a patient's medication when the six-minute walk test shows notable changes in the time it takes for the patient to complete the walk test.

At 902, the processor also determines whether the risk weighted composite index exceeds one or more thresholds. When the risk weighted composite index does not exceed the threshold(s), the process interprets the condition as an indication that the incoming HR data, accelerometer data and/or IMD data indicates that a patient's health condition remains relatively stable. This is assessed as either an instantaneous change relative to the last risk weighted composite index, or based on a gradual increase in the risk weighted composite index over a pre-defined period of time. Accordingly, flow moves to 904 where the process determines that no other action is necessary. In some cases, the data is retained in the patient network and can be reviewed by a medical practitioner, such as by displaying, on a display, graphical representations, such as those of Figures 3 and 4A-4F, and/or comparing data with templates. Alternatively, when the risk weighted composite index exceeds the threshold, the process interprets the condition as an indication that the incoming HR data, accelerometer data and/or IMD data indicates that a patient's health condition is deteriorating and in connection there with flow moves to 906.

At 906, the processor generates a treatment notification based on the treatment diagnosis and directs the treatment notification to be sent to the patient and/or a care provider. At 908, the one or more processors determine whether a change in care has been identified by the treatment diagnosis. Optionally, the operation at 908 may be implemented manually by a clinician or other medical practitioner. As a further option, the operation at 908 may be implemented automatically by one or more processors, as well as manually by a clinician or medical practitioner. The clinician or medical practitioner may then be afforded an option to "override" or modify the automated determination of a change in care. In some embodiments, the medical practitioner can display, on a display, graphical representations, such as those of Figures 3 and 4A-4F, and/or comparisons of the data with templates.

At 910, the processor determines whether to obtain additional HR data, accelerometer data and/or IMD data and the process continues by obtaining additional data. For example, the operation at 910 may simply represent a continuous loop at which the healthcare system 820 waits to receive new/additional HR data, accelerometer data and/or IMD data. Additionally, or alternatively, the processor may determine (e.g., as part of the treatment diagnosis) that further data should be obtained before a change in care is decided. Optionally, the operation at 910 may be implemented manually by a clinician or other medical practitioner. As a further option, the operation at 910 may be implemented automatically by one or more processors, as well as manually by a clinician or medical practitioner. The clinician or medical practitioner may then be afforded an option to "override" or modify the automated determination to obtain additional HR data, accelerometer data and/or IMD data. The process of Figure 9 automatically develops the treatment diagnosis (e.g., clinical insights) based on all available data. The clinical insights may result in a determination to i) obtain more data, ii) recommend a change in clinical care or otherwise. Optionally, a clinician may be afforded the option to "Opt-In" or "Opt-out" of one or more different features and applications, thereby allowing the clinicians to choose which clinical insights they receive in connection with managing patients. Optionally, a clinician may be afforded the option to make decisions (e.g., render a treatment diagnosis, change treatment, obtain more data) and/or validate/reject decisions rendered automatically by the one or more processors.

Optionally, the process of Figure 9 may be implemented in whole or in part within one or more accelerometer circuit, IMD, a PDE device, and/or a local external computing device. For example, an IMD may track the IMD data (e.g., HR and/or accelerometer) and detect possible deterioration of patient's health. When the IMD detects possible deterioration, the IMD may automatically obtain or generate accelerometer data (when the accelerometer circuit is in the IMD). Additionally, or alternatively, the IMD may instruct another IMD, a dedicated accelerometer circuit or other device to obtain or generate accelerometer data. Additionally, or alternatively, the IMD may instruct the patient to perform a prescribed physical activity and/or initiate another action in connection with obtaining supplemental accelerometer data. The combination of the IMD data, HR data, and the accelerometer data is analyzed in accordance with embodiments herein locally or remotely. Additionally, or alternatively, the accelerometer may perform continuous monitoring for indications of possible deterioration in a patient condition. When the accelerometer identifies a possible deterioration in a patient condition, the accelerometer may automatically convey a device command to an IMD to direct the IMD to obtain supplemental IMD data. The combination of the accelerometer data and the supplemental IMD data is analyzed in accordance with embodiments herein locally or remotely.

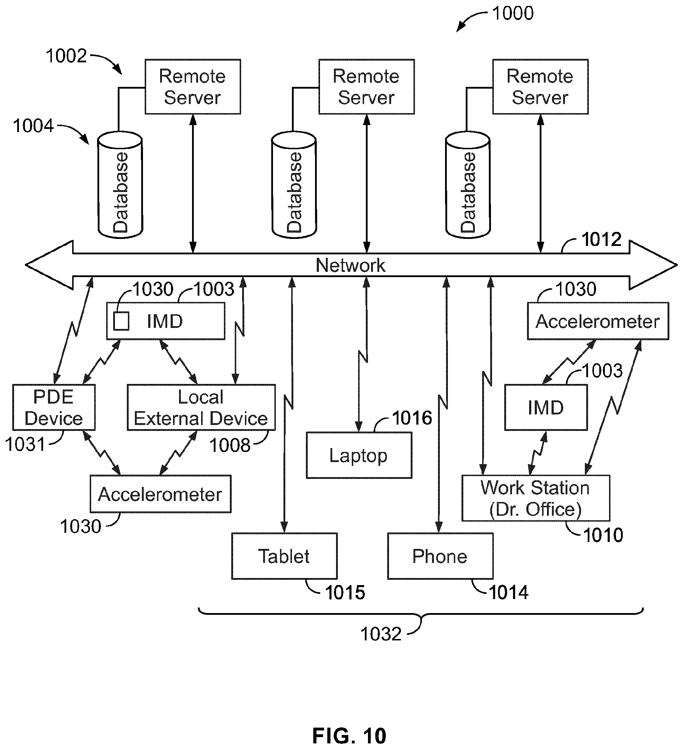

Figure 10 illustrates a healthcare system 1000 formed in accordance with embodiments herein. The healthcare system 1000 includes one or more servers 1002, each of which is connected to one or more database 1004. The servers 1002 and databases 1004 may be located at a common physical location and/or distributed between multiple remote locations within a city, state, country or worldwide. The servers 1002 communicate with one or more networks 1012. The network 1012 may be the internet, a voice over IP (VoIP) gateway, a local plain old telephone service (POTS) such as a public switched telephone network (PSTN), a cellular phone-based network, and the like. Alternatively, the network 1012 may be a local area network (LAN), a campus area network (CAN), a metropolitan area network (MAN), or a wide area network (WAM). The network 1012 serves to provide a network that facilitates the transfer/receipt of information such as IMD data, HR data and accelerometer data.

The system 1000 also includes one or more IMDs 1003, one or more local external devices 1008, one or more accelerometer 1030 (separate or housed within an IMD 1003) detecting accelerometer signals indicative of acceleration, activity level and/or posture, one or more patient data entry (PDE) devices 1031 and one or more medical personnel (MP) devices 1032, all of which communicate (directly or indirectly) through the network 1012 to the servers 1002 and/or one another. The IMD 1003 may be passive or active, may obtain various types of data, such as cardiac electrical and/or mechanical activity data, HR, PAP or other pressure related data, impedance data, RPM data, flow data, and the like. For example, the IMD 1003 and accelerometer 1030 can perform any of the activities, actions, monitoring, analysis, etc., described herein. The PDE device 1031 may communicate with any or all of the IMDs 1003, local external devices 1008, an accelerometer 1030, as well as the network 1012. The PDE device 1031 obtains behavior related medical data (BRM data), such as based on manual inputs from a patient or other user, and/or based on automatic video and/or audio monitoring.

The local external device 1008 may be implemented as a variety of devices including, but not limited to, medical personnel programmer, a local RF transceiver and a user workstation, smart phone, tablet device, laptop computer, desktop computer and the like, and can display graphical representations, diagnosis, treatment recommendations, and the like as discussed herein. The MP devices 1032 may also be implemented as a variety of devices including, but not limited to, medical personnel programmer, workstation, smart phone, tablet device, laptop computer, desktop computer and the like. Functionality of the MP device 1032 related to embodiments herein may be implemented through dedicated hardware circuits, firmware, software, and/or application(s) operating on one or more computing devices. It should be understood that each of the external computing devices (e.g. local external device 1008, workstation 1010, phone 1014, tablet 1015, laptop 1016, PDE device 1031) can include and/or communicate with a display (not shown) for displaying graphical representations, treatment recommendations, diagnosis, and the like as described herein.

The server 1002 is a computer system that provides services to other computing systems over a computer network. The servers 1002 control the communication of information including IMD data, patient entered data, medical record information and accelerometer data. The servers 1002 interface with the network 1012 to transmit/transfer information between the servers 1002, databases 1004, local external devices 1008, medical personnel devices 1032 for storage, retrieval, data collection, data analysis, treatment diagnosis, treatment recommendations and the like.

The databases 1004 store all or various portions of the information described herein, including, but not limited to, IMD data, HR data, templates associated with particular diagnosis, accelerometer data, medical record information, treatment diagnoses and recommendations, and the like. Various portions of the information may be downloaded or uploaded in combination or separately to/from the databases 1004, local external devices 1008 and MP devices 1032. The local external device 1008 may reside in a patient's home, a hospital, or a physician's office. The local external device 1008 communicates wired or wirelessly with one or more IMD 1003 and/or accelerometer 1030. The servers and devices described herein may wirelessly communicate with one another utilizing various protocols, such as Bluetooth, GSM, infrared wireless LANs, HIPERLAN, 3G, satellite, as well as circuit and packet data protocols, and the like. Alternatively, a hard-wired connection may be used to connect the servers and devices. The local external device 1008, when implemented as a programmer, may be configured to acquire cardiac signals from the surface of a person (e.g., ECGs), and/or intra-cardiac electrogram (e.g., IEGM) signals from the IMD 1003. The local external device 1008 interfaces with the network 1012 to upload the data and other information to the server 1002.

Optionally, the local external device may represent a local RF transceiver that interfaces with the network 1012 to upload IMD data, HR data, and/or accelerometer data.

The workstation 1010 may interface with the network 1012 via the internet or POTS to download various data, information, diagnoses, and treatment recommendations from the database 1004. Alternatively, the workstation 1010 may download raw data from the surface ECG units, leads, or monitoring device (e.g., IMD 1003) via either the programmer or the local RF transceiver. Once the user workstation 1010 has downloaded the cardiac signal waveforms, ventricular and atrial heart rates, or detection thresholds, the user workstation 1010 may process the information in accordance with one or more of the operations described above. The system may download information and notifications to the cell phone 1014, the tablet device 1015, the laptop 1016, or to the server 1002 to be stored on the database 1004.

Thus, provided is a distributed "digital" healthcare system that obtains various types of data, enables the data to be analyzed by various computing devices within the system and determines one or more treatment diagnosis and treatment recommendation substantially in real-time with the collection of new data. In some cases, treatment can be determined and provided automatically by systems and devices described herein, such as by one IMD or a first and second IMDs that communicate with each other and/or an external device. In this manner, unneeded and undesired hospitalizations may be avoided through preventative detection, reducing costs associated with emergency medical procedures. Additionally, such a system also assists in prolonging a human's life and increases patient care. Thus, an improved system and methodology are provided.

The processor determines a treatment diagnosis based on the HR data and accelerometer data, such as activity level and/or posture. Optionally, the processor may classify the treatment diagnosis based on various predetermined criteria. The processor updates a patient medical record with the HR and accelerometer data. Optionally, the processor may update the patient medical record to include historic information as appropriate based on patient history. Thereafter, the method may terminate without further action and/or, flow may move to one or more of the processes described herein for applying an application-specific model in connection with providing a treatment diagnosis and treatment recommendation. Embodiments herein may implement one or more of the methods and systems as described in U.S. Patent 9,760,679, issuing on September 12, 2017, and titled "Data synchronization between two or more analyte detecting devices in a database".

As explained herein, treatment diagnoses and recommendations may relate to changes in prescriptions, changes in parameters 764 of an IMD 100, 101 and the like. Additionally, treatment diagnoses and recommendations may be implemented in connection with digital health/patient application features that are communicated to the patient through smart phone or another electronic device. The treatment notification may be provided to the patient and/or a caregiver, with the treatment notification representing educational material and/or feedback regarding negative health consequences of a patient's lifestyle choices when the combined data (BRM, IMD, HR and/or accelerometer data) indicates trends in a negative direction. Similarly, the treatment notification may provide feedback regarding positive health consequences of a patient's lifestyle choices when the combined data indicates trends in a positive direction. As another example, when a patient with an IMD and accelerometer avoids exercise for a period of time, the notification may inform a patient that the patient's lack of exercise increases the likelihood of heart failure.

### Closing

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described and illustrated herein, it should be understood that they are provided merely in illustrative and non-restrictive fashion, and furthermore can be regarded as but mere examples of possible working environments in which one or more arrangements or processes may function or operate.

As will be appreciated by one skilled in the art, various aspects may be embodied as a system, method, or computer (device) program product. Accordingly, aspects may take the form of an entirely hardware embodiment or an embodiment including hardware and software that may all generally be referred to herein as a "circuit," "module" or "system." Furthermore, aspects may take the form of a computer (device) program product embodied in one or more computer (device) readable storage medium(s) having computer (device) readable program code embodied thereon.

Any combination of one or more non-signal computer (device) readable medium(s) may be utilized. The non-signal medium may be a storage medium. A storage medium may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random access memory (RAM), a dynamic random access memory (DRAM), a read-only memory (ROM), an erasable programmable read-only memory (EPROM or Flash memory), a portable compact disc read-only memory (CD-ROM), an optical storage device, a magnetic storage device, or any suitable combination of the foregoing.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of network, including a local area network (LAN) or a wide area network (WAN), or the connection may be made through other devices (for example, through the Internet using an Internet Service Provider) or through a hard wire connection, such as over a USB connection. For example, a server having a first processor, a network interface, and a storage device for storing code may store the program code for carrying out the operations and provide this code through its network interface via a network to a second device having a second processor for execution of the code on the second device.

Aspects are described herein with reference to the figures, which illustrate example methods, devices, and program products according to various example embodiments. The program instructions may be provided to a processor of a general purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

The units/modules/applications herein may include any processor-based or microprocessor-based system including systems using microcontrollers, reduced instruction set computers (RISC), application specific integrated circuits (ASICs), field-programmable gate arrays (FPGAs), logic circuits, and any other circuit or processor capable of executing the functions described herein. Additionally, or alternatively, the modules/controllers herein may represent circuit modules that may be implemented as hardware with associated instructions (for example, software stored on a tangible and non-transitory computer readable storage medium, such as a computer hard drive, ROM, RAM, or the like) that perform the operations described herein. The above examples are exemplary only, and are thus not intended to limit in any way the definition and/or meaning of the term "controller." The units/modules/applications herein may execute a set of instructions that are stored in one or more storage elements, in order to process data. The storage elements may also store data or other information as desired or needed. The storage element may be in the form of an information source or a physical memory element within the modules/controllers herein. The set of instructions may include various commands that instruct the modules/applications herein to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. In addition, many modifications may be made to adapt a particular situation or material to the teachings herein without departing from its scope. While the dimensions, types of materials and coatings described herein are intended to define various parameters, they are by no means limiting and are illustrative in nature. Many other embodiments will be apparent to those of skill in the art upon reviewing the above description. The scope of the embodiments should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects or order of execution on their acts.

## Claims

1. A system (1000) comprising:
an implantable medical device, IMD, (100, 700, 1003) configured to be implanted in a patient, the IMD (100, 700, 1003) comprising:
an accelerometer (112, 770, 1030) configured to output an accelerometer signal;
sensing circuitry (744) configured to sense a cardiac activity (CA) signal over a plurality of cardiac cycles;
memory (760) configured to store program instructions; and
one or more processors (721) coupled to the accelerometer (112, 770, 1030) and the sensing circuitry (744) that, when executing the program instructions, are configured to:
determine a heart rate (HR) at a point in time based on the CA signal;
determine an activity level (AL) at the point in time based on accelerometer data, the accelerometer data based on the accelerometer signal, the HR and AL forming an HR-AL event;
compare the HR-AL event to a heart condition (HC) metric that defines combinations of HRs and ALs representing a physiologically normal heart condition and at least one physiologically abnormal heart condition; and
identify a physiologically abnormal heart condition based on the comparison.

2. The system of claim 1, wherein the one or more processors (721) are further configured to:
repeat the determining and comparing operations to build an HC assessment data set;
save the HC assessment data set in the memory (760) of the IMD (100, 700, 1030); and
transmit the HC assessment data set to an external device (110, 754, 1008) or a second IMD (101) configured to be implanted in the patient, the HC assessment data set utilized to identify the physiologically abnormal heart condition.

3. The system of claim 2, further comprising the second IMD (101) configured to deliver therapy to treat the physiologically abnormal heart condition identified by the HC assessment data set.

4. The IMD of any one of claims 1 to 3, further comprising a pulse generator configured to generate electrical pulses to be applied to the patient's heart, wherein the one or more processors (721) are, when executing the program instructions, configured to control the pulse generator to generate pacing pulses to treat the identified physiologically abnormal heart condition.

5. The system of any one of claims 2 to 4, further comprising the external device (110, 754, 1008) configured to receive the HC assessment data set and map the HC assessment data set onto an HR-AL template to form a HC treatment profile, the external device (110, 754, 1008) having a display configured to display a graphical representation of the HC treatment profile.

6. The system of claim 5, wherein the HC treatment profile is divided into activity zones for i) no activity, ii) low activity, iii) moderate activity, and iv) high activity, and further divided into HR zones for i) low HR, ii) medium HR, and iii) high HR, the HC assessment data set mapped into the activity and HR zones.

7. The system of claim 4 or 5, wherein the HC treatment profile indicates an increase in a number of the HR-AL events at a relatively higher HR over at least two activity levels.

8. The system of any one of claims 1 to 7, wherein the HC metric includes upper and lower HC boundaries, wherein a range between the upper and lower HC boundaries corresponds to physiologically healthy HR-AL events, and the regions above the upper HC boundary and below the lower HC boundary correspond to physiologically unhealthy HR-AL events.

9. The system of any one of claims 2 to 8, wherein the one or more processors (721) is further configured to delete the HR-AL events that fall within a range of the HC metric corresponding to the combinations of HRs and ALs representing the physiologically normal heart condition such that the HC assessment data set excludes the HR-AL events that fall within the range.

10. The system of any one of claims 2 to 9, wherein the one or more processors (721) is further configured to save, in the HC assessment data set, the HR-AL events that fall in regions of the HC metric corresponding to the combinations of HRs and ALs representing the physiologically abnormal heart condition.

11. The system of any one of claims 2 to 10, wherein in response to the physiologically abnormal heart condition being identified, the HC assessment data set prescribes treatment for at least one of i) chronotropic incompetence, ii) sick sinus syndrome, iii) heart failure, iv) sleep apnea, or v) fitness level of the patient.

12. The system of any one of claims 2 to 11, wherein the HC assessment data set prescribes no treatment when the HC assessment data set is indicative of the physiologically normal heart condition.

13. The system of any one of claims 2 to 12, further comprising:
wherein the one or more processors (721) is further configured to determine, using the accelerometer data, a posture from within a plurality of postures over the monitoring period; and
wherein each of the HR-AL events saved in the HC assessment data set in the memory (760) is associated with the corresponding posture at the corresponding point in time.

14. The system of claim 13, wherein the HC assessment data set excludes at least a portion of the HR-AL events associated with at least one of the postures.

15. The system of any one of claims 1 to 14, wherein the one or more processors (721) is further configured to transmit the HR-AL event or a result of the comparison to a second device (101, 110, 754, 1008).
